# EUROPEAN PATENT APPLICATION

(11) **EP 4 534 675 A1**
(43) Date of publication of application: **09.04.2025**
(21) Application number: 23811120.7
(22) Date of filing: 25.05.2023
(51) Int. Cl.: C12N 15/62, C12N 15/82, C12N 15/113, A01H 5/00

(54) **METHOD FOR SITE-SPECIFIC INSERTION OF EXOGENOUS SEQUENCE IN GENOME**

(30) Priority: 25.05.2022 CN 202210580767; 06.04.2023 CN 202310363943
(71) Applicant: INSTITUTE OF GENETICS AND DEVELOPMENTAL BIOLOGY, CHINESE ACADEMY OF SCIENCES, Chaoyang District Beijing 100101 (CN)
(72) Inventor: GAO, Caixia, Beijing 100101 (CN); SUN, Chao, Beijing 100101 (CN)
(74) Representative: Michalski Hüttermann & Partner Patentanwälte mbB
(86) International application number: PCT/CN2023/096198
(87) International publication number: WO 2023/227050

(57) **Abstract**

The invention belongs to the field of genetic engineering. Specifically, the present invention relates to a method for site-directed insertion of exogenous sequence in a genome. Specifically, based on the prime editing system (PE), the present invention uses two adjacent pegRNAs with partially overlapping sequences on the reverse transcription template to achieve efficient and precise site-directed insertion of exogenous sequences in the genome, especially genome of a plant. The system is further coupled with a recombinase system such as Cre/Lox or FLP/FRT, etc., to achieve site-directed insertion of large fragment of exogenous sequences in the genome, especially genome of a plant.

## Description

This application claims the priority of the Chinese patent application with application number 202210580767.0 filed on May 25, 2022 and the Chinese patent application with application number 202310363943.X filed on April 6, 2023.

### Technical filed

The invention belongs to the field of genetic engineering. Specifically, the present invention relates to a method for site-directed insertion of exogenous sequence in a genome. Specifically, based on the prime editing system (PE), the present invention uses two adjacent pegRNAs with partially overlapping sequences on the reverse transcription template to achieve efficient and precise site-directed insertion of exogenous sequences in the genome, especially genome of a plant. The system is further coupled with a recombinase system such as Cre/Lox or FLP/FRT, etc., to achieve site-directed insertion of large fragment of exogenous sequences in the genome, especially genome of a plant.

### Background

The rapid development of DNA sequencing technology has brought the field of life sciences into the genome era. The emergence of technologies represented by GWAS has greatly promoted the development of genetics, especially the analysis of many key gene functions in plants, which has great impact on the development of molecular crop breeding. Traditional crop breeding methods represented by hybridization and backcrossing are not enough to support the rapid growth of crop breeding due to time-consuming and labor-intensive reasons. Therefore, the development of new molecular breeding technologies is becoming more and more important.

Transgenic technology has been quickly applied to plant molecular breeding for its ability to quickly and efficiently obtain excellent traits, but it is subject to strict regulation due to its characteristics of introducing foreign genes. In contrast, genome editing technology can precisely modify functional genes at specific sites without introduction of foreign genes, so as to obtain excellent traits more quickly and efficiently. At present, plant genome editing tools mainly include three categories, one is zinc finger nuclease (ZFN); the other is transcription-activating effector-like nuclease (TALEN); the third is clustered regularly spaced short palindromic repeats and their related proteins (CRISPR/Cas). Among them, the CRISPR/Cas system is the most convenient and efficient, and has made great contributions to genetics research and plant molecular breeding in recent years.

The currently widely used CRISPR/Cas system includes an artificially designed single-stranded guide RNA (sgRNA) and a site-specific nuclease Cas9. sgRNA targets genomic DNA at a specific location through the principle of complementary base pairing, and Cas9 and sgRNA form a ribonucleic acid protein complex (RNP) in cells. At the same time, the conformation of Cas9 changes, and a domain (PAM-interaction domain, PI domain) on Cas9 in the complex continuously interacts with the motif NGG (PAM) at various positions on the genome until it finds a location that is complementary to the sgRNA. In the paired position, the RNP complex interacts with DNA to form a new complex. Cas9 unwinds the DNA double strand to form an R-Loop, and at the same time the conformation changes again, and the RuvC and HNH nuclease active domains on it are activated to complete the cleavage of the non-target strand and the target strand respectively to generate a DNA double-strand break (DSB). At this time, the DNA double-strand break will trigger the endogenous DNA repair mechanism of the cell, and it will usually be repaired through the most frequently occurring non-homologous end joining (NHEJ). NHEJ is an error-prone repair pathway, so the insertion or deletion (Indel) of some bases may be randomly introduced near the DSB during the repair process, resulting in the abnormal expression of the gene. In the process of generating DSB, if a piece of exogenous DNA (donor) with homology arms of the genome sequence on both sides of the DSB is provided, the endogenous repair mechanism of the cell may use the donor as a template to perform homologous recombination repair (HR). HR is a precise repair approach that can introduce arbitrary point mutations, fragment insertions, and deletions into the genome. However, this repair pathway occurs very rarely in higher biological cells, especially plant cells, so it has not been widely used. Since then, the base editor (Base editor, BE) based on the CRISPR system has been developed. The BE system uses Cas9 (nCas9-D10A) with an inactivated RuvC domain coupled to a deaminase (cytosine deaminase or adenine deaminase Ammase, corresponding to CBE and ABE), when the RNP complex binds to DNA to form R-Loop, deaminase deaminates cytosine (C) or adenine (A) on the non-target chain to form uracil (U) or hypoxanthine (I), the intracellular repair mechanism will recognize uracil as thymine (T) and hypoxanthine as guanine (G). At this time nCas9 cleaves the target strand, thereby promoting cell generation. The base excision repair pathway (BER) completes C-U-T or A-I-G repair. The BE system can complete efficient and accurate point mutations without relying on the generation of DSB and the HR pathway, so it has been widely used rapidly. The genome editing toolbox of CRISPR systems coupled with other effectors, represented by BE, has also developed rapidly, including coupling transcriptional activators, repressors, or epigenetic modifiers for targeted activation, repression, and epigenetic modification.

Despite the rapid development of the CRISPR molecular toolbox, from simple gene knockout to precise base editing, to transcriptional activation, repression, and epigenetic modification, the targeted and precise insertion of DNA fragments has been difficult to achieve in higher plant cells. The traditional strategy for achieving targeted insertion relies on the generation of DSBs, and when an additional piece of donor DNA without genomic homologous sequences is provided, the donor may be inserted into the vicinity of the DSB through the NHEJ repair pathway after the DSB is generated. However, this process is very imprecise, and the efficiency is also low due to issues such as the way the donor is provided. When an additional piece of donor DNA containing homologous sequences of the genome is provided, the target fragment in the donor may be inserted at the target site by HR repair pathway after the DSB is generated, but the efficiency of this process is extremely low, and it is almost impossible to achieve in higher plant cells.

Due to the low efficiency of HR, site-specific integration of large fragments of DNA can be accomplished with the help of site-specific recombinase (SSR). SSR can specifically recognize and bind a certain DNA sequence (recombination site, RS) and form a synaptonemal complex. A strand exchange process can occur between the two synaptonemal complexes and complete DNA recombination. This process is catalyzed by the SSR activity. The tyrosine or serine residues in the center attack the RS phosphate backbone to cleavage the DNA. After the cleavage, a covalent intermediate is formed and a strand exchange reaction occurs between the two RSs. This process does not require the participation of high-energy cofactors. With the help of the cell's endogenous DNA repair pathway, because it is more efficient. According to the differences in the active center residues of SSR enzymes, it can be divided into tyrosine recombinase family and serine recombinase family. Common tyrosine recombinases include Escherichia coli phage λ integrase, P1 phage Cre recombinase, yeast FLP recombinase, etc., all of which use a conserved tyrosine residue to attack a chain of the RS backbone, exposing the 5' phosphate group and the 3' hydroxyl group, at this time, the 5' phosphate groups of the two RSs are combined with the 3' hydroxyl groups to realize chain exchange, and at the same time, the recombinase bound to the RS is allosteric, attacks the other chain to achieve chain exchange, so as to complete the process of reorganization. Common serine recombinases include Tn3 transposase, Salmonella recombinase Hin, Streptomyces bacteriophage ΦC31 integrase, and mycobacteriophage Bxb1 integrase, etc. The recombination process is similar to that of tyrosine recombinase, except that it uses serine residue to attack the two chains of the RS backbone at the same time, realizing the simultaneous exchange of the two chains of the two RSs, thereby completing the recombination process. SSR has a wide range of applications: it is mainly used as a molecular cloning tool in vitro, and its high efficiency of DNA molecular recombination makes the in vitro molecular cloning of large fragments and multi-fragments very simple; it can be used as a gene or dyeing engineering transformation tool in prokaryotic cells, Deletion, inversion, translocation or integration of large fragments of DNA; in eukaryotic cells of higher organisms, it is mainly used as a tool for deletion of transgenic marker genes. However, it is very difficult to integrate large fragments of DNA at a specific site due to the difficulty of site-specific knock-in of RS.

Recently, a prime editing system (PE) capable of introducing arbitrary base mutations and short DNA insertions and deletions has been developed, and has been widely used in animal and plant genome editing due to its powerful and DSB-independent functions. The prime editing system uses Cas9 (nCas9-H840A) with an inactive HNH domain coupled to a reverse transcriptase (MLV), and simultaneously introduces a reverse transcription template sequence (RT) and a primer binding site(PBS) for the reverse transcriptase at the 3' end of the sgRNA, where the RT has the target mutation sequence and sequences homologous to the genome on both sides of the mutation sequence, and this sgRNA is called pegRNA. After nCas9 cuts the non-target strand, PBS will bind to its 5' end to serve as the initial primer of reverse transcriptase. Then, the reverse transcriptase extends to the 3' end of RT and reverse transcribes the RT sequence into DNA to form a 3' overhang with a mutated sequence, after the cell's endogenous DNA repairing, it is possible to introduce the mutated sequence into the genome, thereby completing any type of genome editing within a certain length.

The efficiency of guide editing systems in higher plant cells is still too low for efficient insertion, and the length of the insertions is very limited. It is speculated that there are three main reasons. First, the frequency of the repair pathway used by the prime editing system in higher plants is relatively low, resulting in a low final editing efficiency; second, RT competes with genomic homologous sequences to bind genomic DNA, hindering reverse transcription; the third is that the reverse transcriptase or pegRNA is easily degraded or the reverse transcription ability is insufficient. There is still a need in the art for systems and methods for efficient insertion of exogenous nucleotide sequences, especially large fragments of exogenous nucleotide sequences, into plant genomes.

### Summary of the invention

In order to avoid the first two reasons for the low efficiency of PE in higher plants, the inventors first designed a double-pegRNA strategy. Two pegRNAs respectively target and bind to the two strands of genomic DNA and there is a certain distance between the PAMs (about 20bp- About 60bp). The RTs of the two pegRNAs only contain the required insertion sequence and the 3' end has a partial overlapping sequence. After the reverse transcription is completed, the two newly synthesized DNA strands are combined and annealed due to the overlapping sequence. The insertion can be completed through a different DNA repair pathways from the original PE system (according to some results of this application, this repair pathway may be SSA, a repair pathway that occurs more frequently in plants).

Recently, the enhanced version of plant-prime editing system (ePPE) established by fusing the retroviral nucleocapsid protein (NC) and deleting the RNaseH active domain of the reverse transcriptase MLV can enhance the ability of reverse transcription or enhance the stability of reverse transcriptase, thereby greatly improving the efficiency of the plant-prime editing system. In addition, adding a secondary structure tevopre at the 3' end of pegRNA (epegRNA) can also enhance the reverse transcription ability or enhance the stability of pegRNA and improve the efficiency of PE.

In order to further improve the insertion efficiency, the inventors used the above-mentioned ePPE system and epegRNA at the same time, thereby realizing efficient site-specific insertion of short fragments in plant somatic cells. At the same time, the DNA integration ability of the Cre/Lox system and FLP/FRT system in the tyrosine recombinase family and the ΦC31 and Bxb1 recombinase systems in the serine family were evaluated in rice somatic cells. It was found that the Cre/Lox system and the FLP/FRT system work better, and so they are combined with the above-mentioned high-efficiency insertion system, and by providing an additional donor of the desired insertion gene with RS, the site-directed insertion of large fragments of foreign nucleotide sequences can be realized in one step.

### Brief description of drawings

Figure 1. Five constructs tested for efficiency of inserting Lox66 or FRT1 using dual pegRNAs in rice protoplasts.
Figure 2. Testing the efficiency of RS insertion using PPE+pegRNA, ePPE+pegRNA, PPE+epegRNA, or ePPE+epegRNA.
Figure 3. Using the ePPE+epegRNA combination to evaluate the relationship between the insertion length (30bp-100bp) and the distance between two pegRNAs (PAM distance 20bp-80bp) and influence of the length of the overlap between the two RTs (10bp-50bp) on the insertion efficiency.
Figure 4. Efficiency of site-directed insertion for NG PAM when Cas9 is replaced by SpG-Cas9 or SpRY-Cas9.
Figure 5. The effect of different promoters of pegRNA on the insertion efficiency.
Figure 6. The effect of using 37 degrees temperature treatment (6B) and the system using MS2-MCP to recruit MLV (6C) on the efficiency of long fragment insertion.

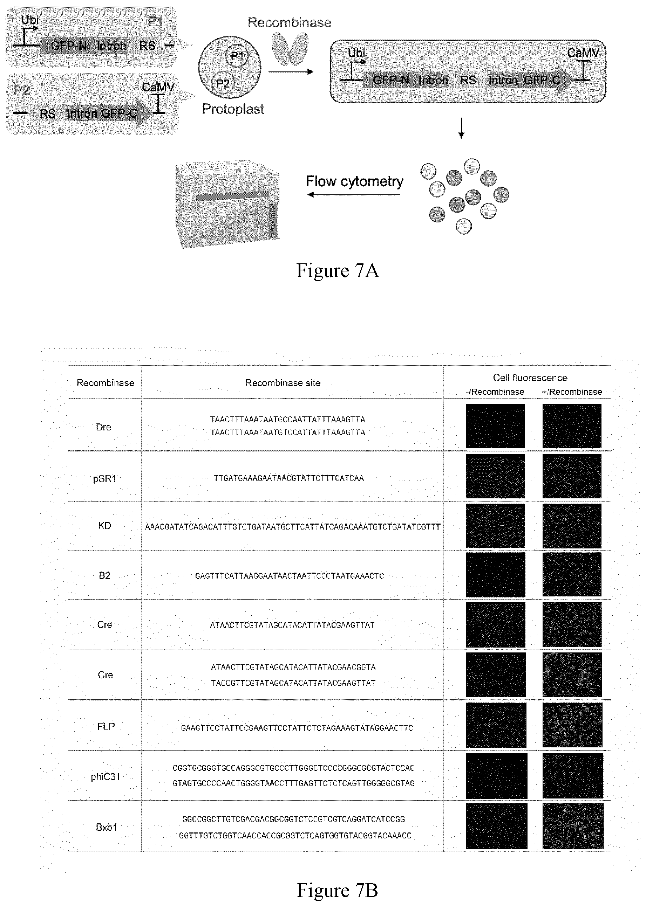

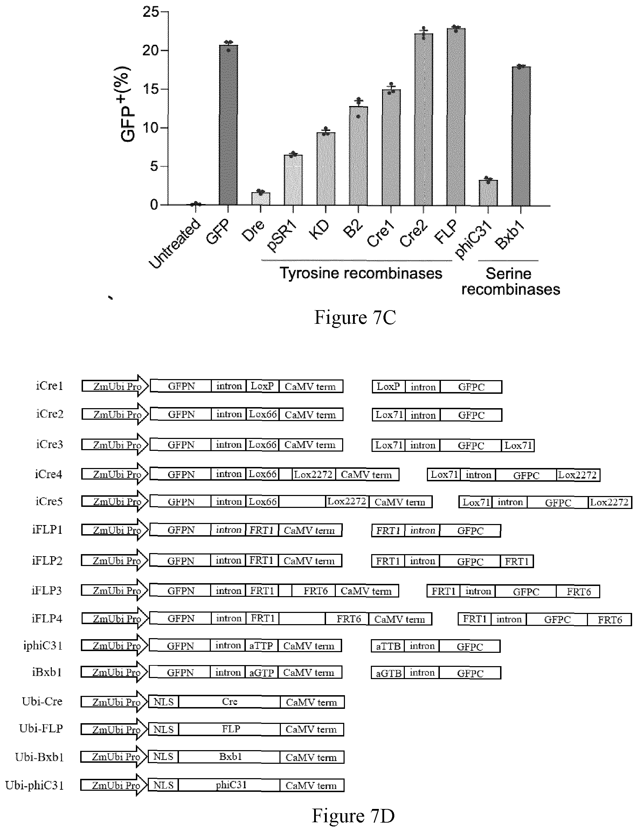

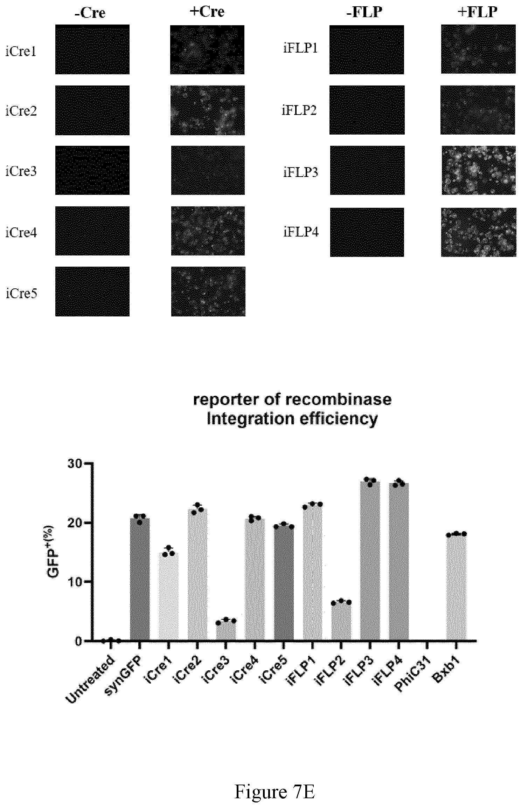

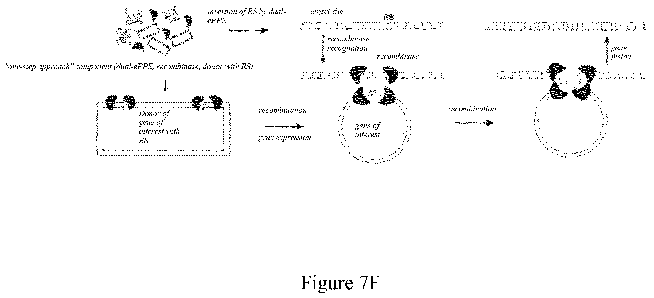

Figure 7. A) Schematic diagram of the GFP reporter system; B) Evaluation of editing effects of 8 recombinases, and the corresponding recombinase site sequences. Microscope images are the rice protoplasts transformed or not with corresponding recombinase; C) Using a fluorescent reporter system to verify the editing effect of the recombinases; D) schematic diagram of the constructs for using a fluorescent reporter system to evaluate the DNA integration ability of the recombinase; F) Schematic representation of constructs for one-step large fragment insertion using recombinase combined with ePPE.
Figure 8. Detection of insertion efficiency of PrimeROOT.v1 system by ddPCR.
Figure 9. A) Percentage of GFP-positive plant protoplast cells by flow cytometry, reflecting the efficiency of "one-step" large fragment insertion using a combination of different recombinases; B) OsALS GFP insertion efficiency in rice protoplast by ddPCR.
Figure 10. Demonstration of the editing efficiency of different editing systems using fluorescence microscopy and flow cytometry.
Figure 11. Using ddPCR to detect the insertion percentage of different donors inserted into the four endogenous sites.
Figure 12. Detection of insertion percentages at six endogenous loci in maize using the PrimeROOT.v2C-Cre system by ddPCR.
Figure 13. Using ddPCR to detect the insertion percentage of different gene editing systems for large fragment insertions.
Figure 14. Comparison of the precise editing efficiency of PrimeROOT.v2C-Cre and NHEJ using base sequencing results.
Figure 15. A) Schematic diagram of the Act1 promoter inserted into the OsHPPD site using PrimeROOT.v2C-Cre; B) screening of pegRNA pairs; C) insertion efficiency.
Figure 16. The GSH site obtained by high-throughput sequencing and the insertion efficiency of the recombination site inserted in GSH1 detected by high-throughput sequencing.
Figure 17. Schematic diagram of PrimeROOT.v3 and efficiency of precision insertion via PrimeROOT.v3.
Figure 18. The efficiency and sequencing results of precise insertion in human HEK293 cells using the PrimeROOT system.

### Detailed description of the invention

### 1. Definition

In the present invention, the scientific and technical terms used herein have the meaning as commonly understood by a person skilled in the art unless otherwise specified. Also, the protein and nucleic acid chemistry, molecular biology, cell and tissue culture, microbiology, immunology related terms, and laboratory procedures used herein are terms and routine steps that are widely used in the corresponding field. For example, standard recombinant DNA and molecular cloning techniques used in the present invention are well known to those skilled in the art and are more fully described in the following document: Sambrook, J., Fritsch, E.F. and Maniatis, T., Molecular Cloning: A Laboratory Manual; Cold Spring Harbor Laboratory Press: Cold Spring Harbor, 1989 (hereinafter referred to as "Sambrook"). In the meantime, in order to better understand the present invention, definitions and explanations of related terms are provided below.

As used herein, the term "and/or" encompasses all combinations of items connected by the term, and each combination should be regarded as individually listed herein. For example, "A and/or B" covers "A", "A and B", and "B". For example, "A, B, and/or C" covers "A", "B", "C", "A and B", "A and C", "B and C", and "A and B and C".

When the term "comprise" is used herein to describe the sequence of a protein or nucleic acid, the protein or nucleic acid may consist of the sequence, or may have additional amino acids or nucleotide at one or both ends of the protein or nucleic acid, but still have the activity described in this invention. In addition, those skilled in the art know that the methionine encoded by the start codon at the N-terminus of the polypeptide will be retained under certain practical conditions (for example, when expressed in a specific expression system), but does not substantially affect the function of the polypeptide. Therefore, when describing the amino acid sequence of specific polypeptide in the specification and claims of the present application, although it may not include the methionine encoded by the start codon at the N-terminus, the sequence containing the methionine is also encompassed, correspondingly, its coding nucleotide sequence may also contain a start codon; vice versa.

As used herein, "genome editing system" refers to the combination of components required for genome editing of the genome within a cell. The various components of the system, such as the guided editing fusion protein or its expression construct, pegRNA or its expression construct, donor construct, etc., can exist independently, or can exist in any combination as a composition.

"Genome" as used herein encompasses not only chromosomal DNA present in the nucleus, but also organelle DNA present in subcellular components of the cell (eg, mitochondria, plastids).

A "genetically modified plant" as used herein means a plant comprising an inserted exogenous polynucleotide within its genome. For example, exogenous polynucleotides can be stably integrated into the genome of a plant and inherited for successive generations. "Exogenous" in reference to a sequence means a sequence from a foreign species, or refers to a sequence in which significant changes in composition and / or locus occur from its native form through deliberate human intervention if from the same species.

"Polynucleotide", "nucleic acid sequence", "nucleotide sequence" or "nucleic acid fragment" are used interchangeably and are single-stranded or double-stranded RNA or DNA polymers, optionally containing synthetic, non-natural or altered nucleotide bases. Nucleotides are referred to by their single letter names as follows: "A" is adenosine or deoxyadenosine (corresponding to RNA or DNA, respectively), "C" means cytidine or deoxycytidine, "G" means guanosine or deoxyguanosine, "U" represents uridine, "T" means deoxythymidine, "R" means purine (A or G), "Y" means pyrimidine (C or T), "K" means G or T, "H" means A or C or T, "I" means inosine, and "N" means any nucleotide. Although nucleotide sequences herein may be expressed as DNA sequences (comprising T), when referring to RNA, one skilled in the art can readily determine the corresponding RNA sequences (i.e., by replacing T with U).

"Polypeptide," "peptide," and "protein" are used interchangeably in the present invention to refer to a polymer of amino acid residues. The terms apply to an amino acid polymer in which one or more amino acid residues is artificial chemical analogue of corresponding naturally occurring amino acid(s), as well as to a naturally occurring amino acid polymer. The terms "polypeptide," "peptide," "amino acid sequence," and "protein" may also include modified forms including, but not limited to, glycosylation, lipid ligation, sulfation, γ carboxylation of glutamic acid residues, and ADP-ribosylation.

As used in the present invention, "expression construct" refers to a vector such as a recombinant vector that is suitable for expression of a nucleotide sequence of interest in a plant. "Expression" refers to the production of a functional product. For example, expression of a nucleotide sequence may refer to the transcription of a nucleotide sequence (eg, transcription to produce mRNA or functional RNA) and / or the translation of an RNA into a precursor or mature protein.

The "expression construct" of the present invention may be a linear nucleic acid fragment, a circular plasmid, a viral vector or, in some embodiments, an RNA that is capable of translation (such as mRNA), such as an RNA generated by in vitro trascription.

The "expression construct" of the present invention may comprise regulatory sequences and nucleotide sequences of interest from different origins, or regulatory sequences and nucleotide sequences of interest from the same source but arranged in a manner different from that normally occurring in nature.

"Promoter" refers to a nucleic acid fragment capable of controlling the transcription of another nucleic acid fragment. In some embodiments of the present invention, the promoter is a promoter capable of controlling the transcription of a gene in a cell, whether or not it is derived from the cell. The promoter may be a constitutive promoter or tissue-specific promoter or developmentally-regulated promoter or inducible promoter.

Examples of promoters include, but are not limited to, the polymerase (pol) I, pol II or pol III promoters. Promoters that can be used in plants include, but are not limited to, cauliflower mosaic virus 35S promoter, maize Ubi-1 promoter, wheat U6 promoter, rice U3 promoter, and rice actin promoter, and the like.

"Introduction" of a nucleic acid molecule (e.g., plasmid, linear nucleic acid fragment, RNA, etc.) or protein into an organism means that the nucleic acid or protein is used to transform a cell of the organism such that the nucleic acid or protein is capable of functioning in the cell. As used in the present invention, "transformation" includes both stable and transient transformations. "Stable transformation" refers to the introduction of exogenous nucleotide sequences into the genome, resulting in the stable inheritance of foreign genes. Once stably transformed, the exogenous nucleic acid sequence is stably integrated into the genome of the organism and any of its successive generations. "Transient transformation" refers to the introduction of a nucleic acid molecule or protein into a cell, performing a function without the stable inheritance of an exogenous gene. In transient transformation, the exogenous nucleic acid sequences are not integrated into the genome.

"Trait" refers to a physiological, morphological, biochemical or physical characteristic of a cell or organism.

"Agronomic traits" specifically refer to measurable indicator parameters of crop plants, including but not limited to: leaf greenness, grain yield, growth rate, total biomass or accumulation rate, fresh weight at maturity, dry weight at maturity, fruit yield, seed yield, plant total nitrogen content, fruit nitrogen content, seed nitrogen content, plant vegetative tissue nitrogen content, plant total free amino acid content, fruit free amino acid content, seed free amino acid content, plant vegetative tissue free amino acid content, total plant protein content, fruit protein content, seed protein content, plant vegetative tissue protein content, herbicide resistance and drought resistance, nitrogen absorption, root lodging, harvest index, stem lodging, plant height, ear height, ear length, disease resistance, cold resistance, salt resistance and tiller number.

### 2. Genome editing system for site-directed modification of the genome of an organism, such as site-specific insertion of an exogenous nucleotide sequence

In one aspect, the present invention relates to a genome editing system for site-directed modification of the genome of an organism, such as site-directed insertion of an exogenous nucleotide sequence, comprising:
i) a) a CRISPR nuclease and/or an expression construct comprising a nucleotide sequence encoding said CRISPR nuclease, and a reverse transcriptase and/or an expression construct comprising a nucleotide sequence encoding said reverse transcriptase, or
   b) a prime editing fusion protein and/or an expression construct comprising a nucleotide sequence encoding the prime editing fusion protein, wherein the prime editing fusion protein comprises a CRISPR nuclease and a reverse transcriptase;
ii) a first pegRNA and/or an expression construct comprising a nucleotide sequence encoding said first pegRNA, and
iii) a second pegRNA and/or an expression construct containing a nucleotide sequence encoding said second pegRNA,

wherein the first pegRNA comprises a first prime sequence, a first scaffold (scaffold) sequence, a first reverse transcription template (RT) sequence and a first primer binding site (PBS) sequence from 5' to 3' direction,
wherein the second pegRNA comprises a second prime sequence, a first scaffold (scaffold) sequence, a second reverse transcription template (RT) sequence and a second primer binding site (PBS) sequence from 5' to 3' direction,
wherein the first pegRNA targets a first target sequence on the sense strand of the genome DNA of the organism, and the second pegRNA targets a second target sequence on the antisense strand of the genome DNA of the organism. In some embodiments, the organism is a plant.

As used herein, "target sequence" refers to a sequence in the genome approximately 20 nucleotides in length characterized by a 5' or 3' flanking PAM (prospacer adjacent motif) sequence. In general, the PAM is required for the recognition of the target sequence by the complex formed by the CRISPR nuclease or its variant and the guide RNA. For example, for the Cas9 nuclease and its variants, the target sequence is immediately adjacent to the PAM at the 3' end, such as 5'-NGG-3'. Based on the presence of PAMs, one skilled in the art can readily determine target sequences in the genome that are available for targeting. And depending on the position of the PAM, the target sequence can be located on any strand of the genomic DNA molecule, and the strand where the target sequence is located is called the target strand. For Cas9 or its derivatives such as Cas9 nickase, the target sequence is preferably 20 nucleotides in length. Depending on the different CRISPR nucleases or their different variants, the PAM sequence may vary.

In some embodiments, the pegRNA is capable of forming a complex with the fusion protein and targeting the fusion protein to a target sequence in the genome, resulting in a nick on the target strand (e.g., within the target sequence).

In some embodiments, the PAMs of the first target sequence and the second target sequence are separated by about 1 to about 300 bp, e.g., 10 bp to about 100 bp, e.g., about 20 bp to about 60 bp. In some embodiments, the PAMs of the first target sequence and the second target sequence may be separated by about 10 bp, about 20 bp, about 30 bp, about 40 bp, about 50 bp, about 60 bp, about 70 bp, about 80 bp, about 100bp, about 150bp, about 300bp.

In some embodiments, the CRISPR nuclease is a Cas9 nuclease, such as SpCas9 derived from S. pyogenes. An exemplary wild-type SpCas9 comprises the amino acid sequence shown in SEQ ID NO:1.

In some embodiments, the CRISPR nuclease is a CRISPR nickase. The CRISPR nickase in the fusion protein is capable of forming a nick within the target sequence on the target strand (the strand on which the target sequence is located) of genomic DNA. In some embodiments, the CRISPR nickase is a Cas9 nickase.

In some embodiments, the Cas9 nickase is derived from SpCas9 of Streptococcus pyogenes (S. pyogenes) and comprises at least the amino acid substitution H840A relative to wild-type SpCas9. In some embodiments, the Cas9 nickase comprises the amino acid sequence shown in SEQ ID NO:2. In some embodiments, the Cas9 nickase can form a nick between the -3 position nucleotide and the -4 position nucleotide of the target sequence (the first nucleotide at the 5' end of the PAM sequence is the +1 position).

In some embodiments, the Cas9 nuclease such as a nickase is a Cas9 nuclease or nickase variant capable of recognizing an altered PAM sequence. Many Cas9 nickase variants capable of recognizing altered PAM sequences are known in the art. In some embodiments, the Cas9 nuclease, such as a nickase, is a Cas9 variant that recognizes the PAM sequence 5'-NG-3'. In some embodiments, the Cas9 nickase variant that recognizes the PAM sequence 5'-NG-3' comprises the following amino acid substitutions H840A, D1135L, S1136W, G1218K, E1219Q, R1335Q, T1337R relative to wild-type Cas9, wherein the amino acid numbering refers to SEQ ID NO:1. In some embodiments, the Cas9 nickase variant (SpG-Cas9 nickase) comprises the amino acid sequence shown in SEQ ID NO:42. In some embodiments, the Cas9 nickase variant that recognizes the PAM sequence 5'-NG-3' comprises the following amino acid substitutions H840A, A61R, L1111R, D1135L, S1136W, G1218K, E1219Q, N1317R, A1322R, R1333P relative to wild-type Cas9 , R1335Q, T1337R, wherein the amino acid numbering refers to SEQ ID NO:1. In some embodiments, the Cas9 nickase variant (SpRY-Cas9 nickase) comprises the amino acid sequence shown in SEQ ID NO:43.

The nick formed by the Cas9 nuclease such as a nickase can cause the target strand to form a free single strand with a 3' end (3' free single strand) and a free single strand with a 5' end (5' free single strand).

In some embodiments, the CRISPR nuclease, such as a Cas9 nickase, and the reverse transcriptase in the prime-editing fusion protein are linked by a linker.

In some embodiments, the reverse transcriptase of the present invention may be derived from different sources. In some embodiments, the reverse transcriptase is a reverse transcriptase of viral origin. For example, in some embodiments, the reverse transcriptase is M-MLV reverse transcriptase or a functional variant thereof. An exemplary wild-type M-MLV reverse transcriptase sequence is shown in SEQ ID NO:3.

In some embodiments, the reverse transcriptase such as M-MLV reverse transcriptase or a functional variant thereof
(a) comprises a mutation at position 155, 156, 200 and/or 524, for example comprises a mutation selected from any one of F155Y, F155V, F156Y, D524N, N200C or a combination thereof, said amino acid position refers to SEQ ID NO: 3;
(b) the connection sequence is deleted; and/or
(c) the RNase H domain is mutated or deleted.

In some preferred embodiments, the reverse transcriptase such as M-MLV reverse transcriptase or a functional variant thereof comprises a mutation D524N, and the amino acid position refers to SEQ ID NO:3.

In some preferred embodiments, the RNase H domain of said reverse transcriptase, e.g., M-MLV reverse transcriptase or a functional variant thereof, is deleted.

In some embodiments, the connection sequence comprises the amino acid sequence shown in SEQ ID NO:4.

In some embodiments, the RNase H domain comprises the amino acid sequence shown in SEQ ID NO:5.

In some embodiments, the reverse transcriptase such as M-MLV reverse transcriptase or a functional variant thereof comprises a sequence shown in any one of SEQ ID NOs:9-15, preferably comprises the amino acid sequence shown in SEQ ID NO: 14.

In some embodiments, the reverse transcriptase, such as M-MLV reverse transcriptase or functional variant thereof, is fused to a nucleocapsid protein (NC), a hydrolase (PR) or an integrase (IN) at the N-terminus or C-terminus directly or via a linker. The nucleocapsid protein (NC), hydrolase (PR) or integrase (IN) is for example from M-MLV

In some embodiments, the nucleocapsid protein (NC) comprises the amino acid sequence shown in SEQ ID NO:6.

In some embodiments, the hydrolase (PR) comprises the amino acid sequence shown in SEQ ID NO:7.

In some embodiments, the integrase (IN) comprises the amino acid sequence shown in SEQ ID NO:8.

In some preferred embodiments, the reverse transcriptase such as M-MLV reverse transcriptase or a functional variant thereof is fused to the nucleocapsid protein (NC) at the N-terminus directly or via a linker.

In some preferred embodiments, the reverse transcriptase such as M-MLV reverse transcriptase or a functional variant thereof is fused to the nucleocapsid protein (NC) at the C-terminus directly or via a linker.

In some embodiments, the reverse transcriptase can also be fused to an RNA aptamer binding protein sequence (such as an MCP protein sequence) via a linker or directly. Thus, the reverse transcriptase can be recruited to the CRISPR nuclease through the interaction of the RNA aptamer binding protein sequence (e.g. MCP protein sequence) and one or more RNA aptamer sequences (e.g. MS2 sequence) present on the pegRNA. In this case, there is no need to fuse the CRISPR nuclease to the reverse transcriptase. An exemplary MCP protein comprises the amino acid sequence of SEQ ID NO:44.

As used herein, a "linker" can be non-functional amino acid sequences without secondary structures, which is 1-50 (e.g., 1, 2, 3, 4, 5, 6, 7, 8, 9, 10, 11, 12, 13, 14, 15, 16, 17, 18, 19, 20 or 20-25, 25-50) or more amino acids in length. For example, the linker can be a flexible linker, such as GGGGS, GS, GAP, (GGGGS)x3, GGS, and (GGS)x7, and the like. For example, it may be the linker shown in SEQ ID NO: 16.

In some embodiments, the CRISPR nuclease, such as a CRISPR nickase, in the fusion protein is located at N-terminal of the reverse transcriptase. In some embodiments, the CRISPR nuclease, such as a CRISPR nickase, in the fusion protein is located at C-terminal of the reverse transcriptase.

In some embodiments of the present invention, the CRISPR nuclease, reverse transcriptase, recombinase or fusion protein of the present invention may further comprise one or more nuclear localization sequences (NLS). Generally, the one or more NLSs in the CRISPR nuclease, reverse transcriptase or fusion protein should be of sufficient strength to drive the CRISPR nuclease, reverse transcriptase or fusion protein in the nucleus of the cell to accumulate in an amount enabling its editing function. In general, the strength of nuclear localization activity is determined by the number, location of NLSs in the CRISPR nuclease, reverse transcriptase or fusion protein, the specific NLS(s) used, or a combination of these factors.

In some preferred embodiments, the fusion protein comprises, from the N-terminus to the C-terminus, the CRISPR nuclease such as nickase, the nucleocapsid protein (NC) and the reverse transcriptase, which are linked with or without a linker . In some preferred embodiments, the fusion protein comprises from the N-terminal to the C-terminal direction, a nuclear localization sequence-the CRISPR nuclease such as nickase-linker-the nucleocapsid protein (NC)-nuclear localization sequence-linker-the reverse transcriptase-nuclear localization sequence.

In some preferred embodiments, the fusion protein comprises the amino acid sequence shown in SEQ ID NO: 19 (ePPE).

In some embodiments, the fusion protein comprises a nuclease moiety comprising the CRISPR nuclease, such as a CRISPR nickase, and one or more NLS, and a reverse transcriptase moiety comprising an RNA aptamer binding protein sequence (e.g. MCP protein sequence), the reverse transcriptase, one or more NLS and optionally the nucleocapsid protein (NC), wherein said nuclease moiety and reverse transcriptase moiety are linked through a self-cleavable peptide. When the fusion protein is translated in vivo, a separate nuclease moiety polypeptide and a reverse transcriptase moiety polypeptide will be formed, and the reverse transcriptase moiety will be recruited to the nuclease moiety through the interaction between the RNA aptamer binding protein sequence (such as the MCP protein sequence) and one or more RNA aptamer sequences (such as MS2 sequences) present on the pegRNA. An exemplary MCP protein comprises the amino acid sequence of SEQ ID NO:44.

In some embodiments, the pegRNA of the invention further comprises one or more RNA aptamer sequences (e.g., MS2 sequences). Exemplary one or more MS2 sequences are set forth in SEQ ID NO:45. In some embodiments, the one or more RNA aptamer sequences (e.g., MS2 sequences) are located at 3' of the pegRNA. In some embodiments, the one or more RNA aptamer sequences (e.g., MS2 sequence) are located in the middle of the pegRNA, e.g., between the scaffold sequence and the RT sequence. The one or more RNA aptamer sequences (e.g., MS2 sequence) can be used to recruit a reverse transcriptase comprising an RNA aptamer binding protein sequence (e.g., MCP protein sequence) to the CRISPR nuclease-pegRNA complex.

The prime sequence (also known as seed sequence or spacer sequence) in the pegRNA of the present invention is set to have sufficient sequence identity (preferably 100% identity) with the target sequence, so that it can bind to the complementary strand of the target sequence through base pairing to achieve sequence-specific targeting.

For example, the prime sequence in the first pegRNA may have sufficient sequence identity (preferably 100% identity) to the first target sequence such that the first pegRNA's complex with a CRISPR nuclease such as a nickase results in a nick in the first target sequence; the prime sequence in the second pegRNA may have sufficient sequence identity (preferably 100% identity) to the second target sequence on the opposite strand such that the second pegRNA's complex with a CRISPR nuclease such as a nickase results in a nick in the second target sequence, thereby the two pegRNAs result in nicks on different strands of the genomic DNA.

A variety of scaffold sequences for gRNAs suitable for genome editing based on CRISPR nucleases (e.g., Cas9) are known in the art, and these can be used in the pegRNAs of the present invention. In some embodiments, the scaffold sequence of the gRNA is shown in SEQ ID NO: 17.

In some embodiments, the primer binding sequence is configured to be complementary to at least a part of the target sequence (preferably perfectly paired with at least a part of the target sequence), preferably, the primer binding sequence is complementary to at least a part of the 3' free single strand resulted from the nick in the DNA chain where the target sequence is located (preferably perfectly paired with at least a part of the 3' free single strand), especially complementary to the nucleotide sequence at the 3' end of the 3' free single strand (preferably perfect pairing). When the 3' free single strand of the chain binds to the primer binding sequence through base pairing, the 3' free single strand can serve as a primer, and the reverse transcription template (RT) sequence immediately adjacent to the primer binding sequence can be used as a template, reverse-transcription is performed under the action of reverse transcriptase to extend a DNA sequence corresponding to the reverse transcription template (RT) sequence.

The primer binding sequence depends on the length of the free single strand formed in the target sequence by the CRISPR nickase as used, however, it should be of the minimum length to ensure specific binding. In some embodiments, the length of the primer binding sequence can be 4-20 nucleotides, for example, the length is 4, 5, 6, 7, 8, 9, 10, 11, 12, 13, 14, 15, 16, 17, 18, 19, or 20 nucleotides.

In some embodiments, the primer binding sequence is configured to have a Tm (melting temperature) of no more than about 52°C. In some embodiments, the Tm (melting temperature) of the primer binding sequence is about 18°C-52°C, preferably about 24°C-36°C, more preferably about 28°C-32°C, more preferably about 30°C.

The method for calculating the Tm of a nucleic acid sequence is well known in the art, for example, it can be calculated using an online analysis tool of Oligo Analysis Tool. An exemplary calculation formula is Tm=N_{G:C}*4+N_{A:T}*2, wherein N_{G:C} is the number of G and C bases in the sequence, and N_{A:T} is the number of A and T bases in the sequence. A suitable Tm can be obtained by selecting a suitable length of PBS. Alternatively, a PBS sequence with an appropriate Tm can be obtained by selecting an appropriate target sequence.

In some embodiments, the RT template sequence can be any sequence. Through the above reverse transcription, its sequence information can be integrated into the DNA strain where the target sequence is located (that is, the strain containing the target sequence PAM), and then through the DNA repair function of the cell, a DNA double strand containing the sequence information of the RT template is formed. In some embodiments, the RT template sequence comprises the desired modification. For example, the desired modification includes substitution, deletion and/or addition of one or more nucleotides. In some embodiments, the RT template sequence is configured to correspond to (e.g., be complementary to at least a portion of) the sequence downstream of the nick of the target sequence but comprise desired modifications. Such desired modification includes substitution, deletion and/or addition of one or more nucleotides.

In some embodiments, the two pegRNAs are configured to introduce a same desired modification. For example, one of the pegRNAs is configured to introduce a substitution from A to G in the sense strand, while the other pegRNA is configured to introduce a substitution from T to C in the corresponding position of the antisense strand. For another example, one of the pegRNAs is configured to introduce a two-nucleotide deletion in the sense strand, and the other pegRNA is configured to also introduce a two-nucleotide deletion in the corresponding position of the antisense strand. Other types of modification can be deduced by analogy. PegRNAs targeting two different strands, respectively, can achieve the same desired modification by designing appropriate RT template sequences.

In some embodiments, the RT sequence is configured to generate an exogenous nucleotide sequence or part thereof to be inserted into the genome after reverse transcription using it as a template, or to generate a complement of the exogenous nucleotide sequence or a portion thereof to be inserted into the genome of an organism such as a plant. In some embodiments, the RT sequence does not comprise genomic sequence adjacent to the target sequence or the complement of genomic sequence adjacent to the target sequence. In some embodiments, the RT sequence does not contain sequence information other than the exogenous nucleotide sequence to be inserted.

In some embodiments, the first RT sequence and the second RT sequence are used to insert a first exogenous nucleotide sequence, for example, to insert a first exogenous nucleotide sequence between the first target sequence and the second target sequence (e.g., between the nick of the first target sequence and the nick of the second target sequence).

In some embodiments, the first RT sequence of the first pegRNA is configured to generate a first fragment of the first exogenous nucleotide sequence to be inserted into the genome after reverse transcription using it as a template; the second RT sequence of the second pegRNA is configured to generate the complementary sequence of a second fragment of the first exogenous nucleotide sequence to be inserted into the genome after reverse transcription using it as a template.

In some embodiments, the first segment and the second segment of the first exogenous nucleotide sequence to be inserted at least partially overlap. In some embodiments, the first fragment and the second fragment overlap by at least about 10 bp to about 50 bp, such as overlap by at least about 10 bp, about 15 bp, about 20 bp, about 25 bp, about 30 bp, about 35 bp, about 40 bp, about 45 bp, about 50 bp. In some embodiments, the first segment and the second segment of the first exogenous nucleotide sequence to be inserted completely overlap.

In some embodiments, the length of the first exogenous nucleotide sequence to be inserted is about 1bp-about 700bp, such as about 10bp, about 20bp, about 30bp, about 40bp, about 50bp, about 60bp, about 70bp, About 80bp, about 90bp, about 100bp, about 150bp, about 200bp, about 250bp, about 300bp, about 350bp, about 400bp, about 450bp, about 500bp, about 600bp, about 700bp, or any value in between.

In some embodiments, the pegRNA further comprises a tevopre sequence at the 3' end of the PBS. The design of tevopre sequence may refer to James W. Nelson et al., Engineered pegRNAs improve prime editing efficiency. 2022, Nature Biotech. volume 40, pages 402-410. An exemplary tevopre sequence is shown in SEQ ID NO:20.

In some embodiments, the pegRNA further comprises a polyA sequence at the 3' end. The polyA sequence comprises, for example, a contiguous sequence of about 10-30 adenine nucleotides (A).

In some embodiments, from the 5' to 3' direction, the pegRNA comprises a guide sequence, a scaffold sequence, a reverse transcription template (RT) sequence, a primer binding site (PBS) sequence, a tevopre sequence, and a polyA sequence.

In some embodiments, the pegRNA can be precisely processed to its sequence using a self-processing system. In some embodiments, the 5' end of the pegRNA is linked to a first ribozyme or tRNA designed to cleave the fusion at the 5' end of the pegRNA; and/ or the 3' end of the pegRNA is linked to a second ribozyme or tRNA designed to cleave the fusion at the 3' end of the pegRNA. The design of the first or second ribozyme or tRNA is within the purview of those skilled in the art. For example, see Gao et al., JIPB, Apr , 2014; Vol 56, Issue 4, 343-349. A method for precisely processing gRNA can be referred to, for example, WO 2018/149418.

In some embodiments, transcription of the first pegRNA and the second pegRNA are driven by different promoters. For example, the first pegRNA is expressed by an OsU3 promoter, and the second pegRNA is expressed by a TaU3 promoter.

In some embodiments, transcription of the pegRNA is driven by a Type II promoter, that is, in an expression construct comprising a nucleotide sequence encoding the pegRNA, the encoding nucleotide sequence of the pegRNA and the Type II promoter are operably linked . In some specific embodiments, the Type II promoter is a GS promoter. The sequence of an exemplary GS promoter is shown in SEQ ID NO:21.

In some embodiments, said first target sequence, second target sequence and/or said desired modification such as said first exogenous nucleotide sequence is associated with a trait (such as an agronomic trait) of an organism such as a plant , whereby said desired modification, such as insertion of a first exogenous nucleotide sequence, results in an altered (preferably improved) trait, such as an agronomic trait, of said organism, such as a plant, relative to a wild-type organism, such as a plant.

In some embodiments, the first exogenous nucleotide sequence comprises one or more recombinase recognition sites (RS).

In some embodiments, the recombinase is a recombinase of the tyrosine recombinase family or a recombinase of the serine recombinase family, preferably a recombinase of the tyrosine recombinase family. Exemplary tyrosine recombinases include, but are not limited to, Escherichia coli phage lambda integrase, P1 bacteriophage Cre recombinase (cyclization recombinase), yeast FLP recombinase (flippase recombinase). Exemplary serine recombinases include, but are not limited to, Tn3 transposase, Salmonella recombinase Hin, Streptomyces bacteriophage ΦC31 integrase, and mycobacteriophage Bxb1 integrase. Different recombinases and their corresponding recombinase recognition sites (RS) are known in the art, and can be selected by those skilled in the art according to needs.

In some embodiments, the recombinase is Dre recombinase. An exemplary Dre recombinase comprises the amino acid sequence of SEQ ID NO:56. Correspondingly, the one or more recombinase recognition sites (RS) include but not limited to rox (SEQ ID NO: 57, 58).

In some embodiments, the recombinase is ΦC31 integrase. An exemplary ΦC31 integrase comprises the amino acid sequence of SEQ ID NO:22. Correspondingly, the one or more recombinase recognition sites (RS) include but are not limited to aTTP (SEQ ID NO: 38) and/or aTTB (SEQ ID NO: 39).

In some preferred embodiments, the recombinase is Bxb 1 integrase. An exemplary Bxb1 integrase comprises the amino acid sequence of SEQ ID NO:23. Correspondingly, the one or more recombinase recognition sites (RS) include but are not limited to aGTP (SEQ ID NO:40) and/or aGTB (SEQ ID NO:41).

In some preferred embodiments, the recombinase is Cre recombinase. An exemplary Cre recombinase comprises the amino acid sequence of SEQ ID NO:24. Correspondingly, the one or more recombinase recognition sites (RS) include but are not limited to loxP (SEQ ID NO:26), Lox2272 (SEQ ID NO:29), Lox71 (SEQ ID NO:27), Lox66 ( SEQ ID NO: 28) or their variants, and any combination thereof.

In some preferred embodiments, the recombinase is FLP recombinase. An exemplary FLP recombinase comprises the amino acid sequence of SEQ ID NO:25. Correspondingly, the one or more recombinase recognition sites (RS) include but are not limited to FRT1 (SEQ ID NO: 30), FRT6 (SEQ ID NO: 31) or their variants, and any combination thereof. In some embodiments, the one or more recombinase recognition sites (RS) are variants of FRT1, e.g., comprising the sequence set forth in one of SEQ ID NOs: 32-37.

In some embodiments, the recombinase is B2 recombinase. An exemplary B2 recombinase comprises the amino acid sequence of SEQ ID NO:50. Correspondingly, the one or more recombinase recognition sites (RS) include but are not limited to the nucleotide sequence shown in SEQ ID NO:53.

In some embodiments, the recombinase is KD recombinase. An exemplary KD recombinase comprises the amino acid sequence of SEQ ID NO:51. Correspondingly, the one or more recombinase recognition sites (RS) include but are not limited to the nucleotide sequence shown in SEQ ID NO:54.

In some embodiments, the recombinase is pSR1 recombinase. An exemplary pSR1 recombinase comprises the amino acid sequence of SEQ ID NO:52. Correspondingly, the one or more recombinase recognition sites (RS) include but are not limited to the nucleotide sequence shown in SEQ ID NO:55.

Based on one or more recombinase recognition sites (RS) in the first exogenous nucleotide sequence inserted into the genome, by providing a donor comprising the RS and a second exogenous nucleotide sequence, using the corresponding recombinase, the second exogenous nucleotide sequence can be inserted into the genome of an organism such as a plant by recombination. The recombinase can be expressed separately, or can be included in the prime editing fusion protein. Those skilled in the art can select a suitable combination of the RS located in the first exogenous polynucleotide inserted into the genome and the RS located in the donor to insert the second exogenous nucleotide sequence into the genome through recombination.

Therefore, in some embodiments, the genome editing system further comprises:
iv) a recombinase and/or an expression construct comprising a nucleotide sequence encoding said recombinase, and
v) a donor construct comprising one or more recombinase recognition sites (RS) and a second exogenous polynucleotide sequence to be inserted into the genome.

In some preferred embodiments, said recombinase is comprised in said prime editing fusion protein. In some embodiments, the recombinase is located at N-terminal of the prime editing fusion protein relative to the CRISPR nuclease and reverse transcriptase. In some embodiments, the recombinase is located at C-terminal of the guide editing fusion protein relative to the CRISPR nuclease and reverse transcriptase.

The second exogenous polynucleotide sequence can be of any length. The second exogenous polynucleotide sequence may be 1 bp to about 10 kb or longer. Preferably, the second exogenous polynucleotide is a long fragment, such as at least 300bp, at least 500bp, at least 1kb, at least 1.5kb, at least 2kb, at least 3kb, at least 4kb, at least 5kb, at least 6kb, at least 7kb, at least 8kb , at least 9kb, at least 10kb or longer. In some embodiments, the second exogenous polynucleotide can be a full-length gene.

In some embodiments, wherein the second exogenous nucleotide sequence is related to a trait such as an agronomic trait of an organism, such as a plant, whereby insertion of the second exogenous nucleotide sequence results in altered (preferably improved) traits, e.g. agronomic traits in the organism, such as a plant, relatively to the wild-type organism such as a plant.

Different components of the genome editing system of the present invention, such as coding sequences of the CRISPR nuclease, the reverse transcriptase, the prime editing fusion protein, pegRNA and/or recombinase, and the second exogenous polynucleotide sequence can be located on a same construct in different combinations, or on separate constructs.

Organisms that can undergo site-directed modification such as site-directed insertion of exogenous nucleotide sequences by the genome editing system of the present invention can be non-human animals, humans or plants, preferably plants. Suitable plants include monocots and dicots, for example, the plants are crop plants including, but not limited to, wheat, rice, corn, soybean, sunflower, sorghum, rape, alfalfa, cotton, barley, millet, sugar cane, tomato, tobacco, cassava and potatoes.

In order to obtain effective expression in organisms such as plants, in some embodiments of the present invention, the nucleotide sequence encoding the fusion protein is codon-optimized for the organisms whose genomes are to be modified, such as plant species.

The codon optimization refers to a method for replacing at least one codon in the natural sequence (for example, about or more than about 1, 2, 3, 4, 5, 10, 15, 20, 25, 50 or more codons) with a codon used more frequently or most frequently in the gene of the host cell, and maintaining the natural amino acid sequence while modifying the nucleic acid sequence to enhance expression in the host cell of interest. Different species exhibit specific preferences for certain codons of specific amino acids. Codon preference (difference in codon usage between organisms) is often related to the translation efficiency of messenger RNA (mRNA), which is considered as depending on the nature of the codon being translated and the availability of the specific transfer RNA (tRNA) molecule. The advantages of the selected tRNA in the cell generally reflect the codons most frequently used for peptide synthesis. Therefore, genes may be tailored to the optimal gene expression in a given organism based on codons optimization. The codon usage tables may be easily obtained, for example, in the codon usage database ("Codon Usage Database") available at www.kazusa.orjp/codon/, and these tables may be adjusted and applied in different ways. See Nakamura Y. et al., "Codon usage tabulated from the international DNA sequence databases: status for the year 2000". Nucl. Acids Res., 28: 292 (2000).

### 3. Method for site-directed modification in plant genome, such as site-directed insertion of exogenous nucleotide sequence

In another aspect, the present invention provides a method for site-directed modification of plant genome, comprising introducing the genome editing system of the present invention into at least one of said plants. The site-directed modification includes substitution, deletion and/or addition of one or more nucleotides. For example, the site-directed modification includes site-directed insertion of an exogenous nucleotide sequence.

In another aspect, the invention provides a method of producing a genetically modified plant comprising a site-directed modification, the method comprising introducing a genome editing system of the invention into at least one of said plants. The site-directed modification includes substitution, deletion and/or addition of one or more nucleotides. For example, the site-directed modification includes site-directed insertion of an exogenous nucleotide sequence.

In some embodiments, the method further comprises screening said at least one plant for a plant having a desired site-directed modification, e.g., site-directed insertion of an exogenous nucleotide sequence.

In the method of the present invention, the genome editing system can be introduced into plant by various methods well known to those skilled in the art. Methods that can be used to introduce the genome editing system of the present invention into a plant include, but are not limited to: biolistic method, PEG-mediated protoplast transformation, Agrobacterium-mediated transformation, plant virus-mediated transformation, pollen tube method, and ovary injection method. Preferably, said genome editing system is introduced into the plant by transient transformation.

In some embodiments, the components of the genome editing system are simultaneously introduced into the plant. In some embodiments, the components of the genome editing system are introduced into the plant separately or sequentially.

In some embodiments, the method comprises the steps of:
1) transforming components i)-iv) of the genome editing system into an isolated plant cell or tissue to obtain a plant cell or tissue inserted with a first exogenous nucleotide sequence comprising one or mor recombinases recognition sites (RS) ;
2) transforming component v) of the genome editing system into the plant cell or tissue obtained in step 1), thereby obtaining a plant cell or tissue comprising the inserted second exogenous polynucleotide sequence; and
3) Regenerating an intact plant from the plant cell or tissue obtained in step 2).

In some embodiments, the exogenous nucleotide sequence is inserted into a safe harbor site in the plant genome, the safe harbor site in the plant genome is
1) at least 5kb away from protein coding region;
2) at least 30kb away from miRNA coding region;
3) at least 20kb away from lncRNA coding region;
4) at least 20kb away from tRNA coding region;
5) at least 5kb away from promoter and/or enhancer;
6) at least 20kb away from LTR repeat;
7) at least 200bp away from non-LTR repeat; and
8) at least 10kb away from the centromere.

In some embodiments, the plant is rice, and the safe harbor sites are selected from the sites shown in Tables 1 and 2.

In some embodiments, the introduction comprises transforming the genome editing system of the invention into an isolated plant cell or tissue, and then regenerating the transformed plant cell or tissue into an intact plant. Preferably, no selection agent for the selection gene carried on the expression vector is used during tissue culture.

In other embodiments, the genome editing system of the present invention can be transformed into a specific part on an intact plant, such as leaves, shoot tips, pollen tubes, young ears or hypocotyls. This is particularly suitable for the transformation of plants that are difficult to regenerate in tissue culture.

In some embodiments of the invention, an in vitro expressed protein and/or an in vitro transcribed RNA molecule (e.g., the expression construct is an in vitro transcribed RNA molecule) and/or a donor DNA molecule is directly transformed into the plant.

In some embodiments, the method further comprises treating (such as culturing) the plant cell, tissue or intact plant that has been introduced with the genome editing system at an elevated temperature (relative to the temperature of conventional cultivation, such as room temperature), the elevated temperature is for example 37°C.

In some embodiments of the present invention, said site-directed modification such as site-directed insertion of an exogenous nucleotide sequence and/or said target sequence is related to a plant trait such as an agronomic traits, whereby said site-directed modification such as site-directed insertion results in altered (preferably improved) traits, such as agronomic traits of said plant relative to a wild-type plant.

In some embodiments, the method further comprises the step of screening for a plant having a desired site-directed modification, such as a site-directed insertion, and/or a desired trait, such as an agronomic trait.

In some embodiments of the invention, the method further comprises obtaining progeny of the genetically modified plant. Preferably, the genetically modified plant or progeny thereof has a desired modification (such as site-directed insertion of an exogenous polynucleotide) and/or a desired trait, such as an agronomic trait.

In another aspect, the present invention also provides a genetically modified plant or a progeny thereof or a part thereof, wherein said plant is obtained by the above-mentioned method of the present invention. Preferably, the genetically modified plant or progeny thereof has a desired genetic modification (such as site-directed insertion of an exogenous polynucleotide) and/or a desired trait, such as an agronomic trait.

In another aspect, the present invention also provides a method of plant breeding, comprising crossing a first genetically modified plant obtained by the above-mentioned method of the present invention with a second plant that does not contain the modification, so that the modification (e.g., site-directed insertion of exogenous polynucleotide) is introduced into the second plant. Preferably, said first genetically modified plant and said second plant have the desired trait such as agronomic trait.

Plants that can undergo site-directed modification such as site-directed insertion of exogenous nucleotide sequences through the genome editing system of the present invention include monocotyledonous plants and dicotyledonous plants. For example, the plants are crop plants, including but not limited to wheat, rice, corn, Soybean, sunflower, sorghum, canola, alfalfa, cotton, barley, millet, sugar cane, tomato, tobacco, cassava and potato.

In another aspect, the present invention provides a method of producing a genetically modified plant comprising a site-directed insertion of an exogenous nucleotide sequence, the method comprising inserting the exogenous nucleotide sequence into a safe harbor site in the genome of the plant, said safe harbor site in said plant genome is
1) at least 5kb away from a protein coding region;
2) atleast 30kb away from a miRNA coding region;
3) atleast 20kb away from lncRNA coding region;
4) atleast 20kb away from a tRNA coding region;
5) atleast 5kb away from a promoter and/or enhancer;
6) at least 20kb away from a LTR repeat;
7) atleast 200bp away from a non-LTR repeat; and
8) atleast 10kb away from the centromere.

In some embodiments, the plant is rice, and the safe harbor site is selected from the sites shown in Tables 1 and 2.

### 4. Methods for site-directed modification in the genome of human or non-human animal , such as site-directed insertion of an exogenous nucleotide sequence

In another aspect, the present invention provides a method for site-directed modification of the genome of human or non-human animal, comprising introducing the genome editing system of the present invention into at least one cell of the human or non-human animal. The site-directed modification includes substitution, deletion and/or addition of one or more nucleotides. For example, the site-directed modification includes site-directed insertion of an exogenous nucleotide sequence.

On the other hand, the present invention provides a gene therapy application of site-directed modification of human or non-human animal genome in vivo or in vitro, which can realize the deletion, addition, up-regulation, down-regulation, inactivation, activation or mutation correction of disease-related genes, etc. Thereby the prevention and/or treatment of diseases is achieved. For example, the target nucleic acid region in the present invention may be located in the protein coding region of a disease-related gene, or may be located in a gene expression regulatory region such as a promoter region or an enhancer region, so that the function of the disease-related gene or disease-associated gene expression can be modified . Therefore, the modification of the disease-related genes described herein includes the modification of the disease-related genes themselves (such as protein coding regions), and also includes the modification of their expression regulation regions (such as promoters, enhancers, introns, etc.).

In another aspect, the present invention provides a method of producing a genetically modified human or non-human animal somatic cell comprising a site-directed modification, the method comprising introducing a genome editing system of the present invention into at least one said human or animal somatic cell. The site-directed modification includes substitution, deletion and/or addition of one or more nucleotides. For example, the site-directed modification includes site-directed insertion of an exogenous nucleotide sequence.

Accordingly, the present invention also provides a method of treating a disease in a subject in need thereof, comprising delivering to the subject an effective amount of the genome editing system of the present invention to modify a gene associated with the disease. The present invention also provides a use of a genome editing system for preparing a pharmaceutical composition for treating a disease in a subject in need thereof, wherein the genome editing system is used to modify a gene associated with the disease. The present invention also provides a pharmaceutical composition for treating a disease in a subject in need thereof, which comprises the genome editing system of the present invention, and optionally a pharmaceutically acceptable carrier, wherein the genome editing system is used to modify the disease-associated genes. In some embodiments, the subject is a human.

### 5. Kit

The present invention also includes a kit for use in the method of the present invention, the kit comprising at least the components of the genome editing system of the present invention. The kit may also include reagents for introducing the genome editing system into an organism or cells of an organism. Kits generally include a label indicating the intended use and/or method of use of the kit contents. The term label includes any written or recorded material provided on or with the kit or otherwise provided with the kit.

### Example

### Example 1. Design of a novel genome editing system

### 1.1. Screening of Prime Editing (PE) System

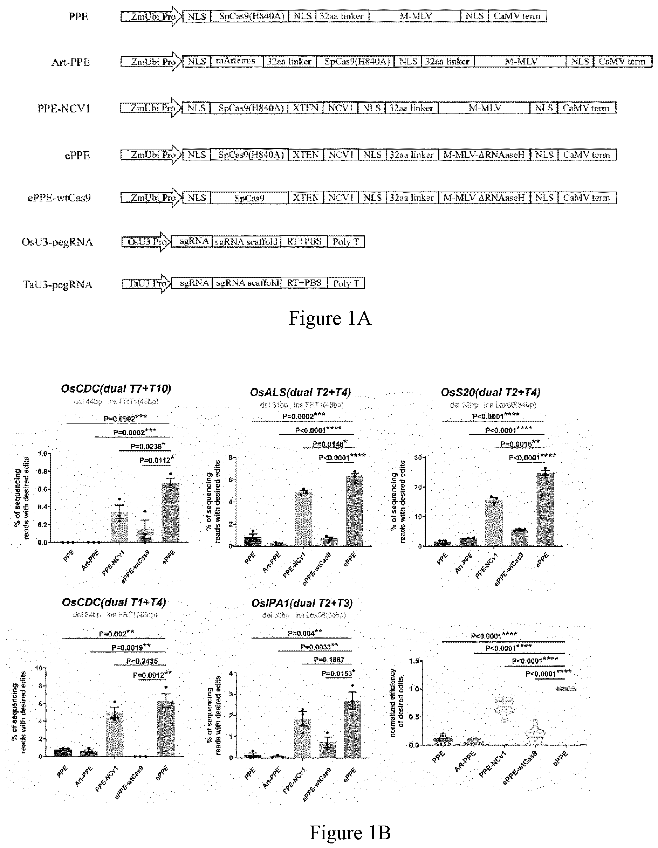

Prime editing (PE) is a precise genome editing technique capable of generating base changes and short DNA insertions and deletions without forming DSBs, and is widely used across species such as human, mouse, rice, wheat, corn etc. In order to develop a novel genome editing system, this example firstly screened the efficiency of the reported PE system for endogenous target editing using the dual pegRNA strategy. Five PE system constructs were compared, namely PPE, Art-PPE (the 5' end of Cas9 fused with mouse exonuclease Artemis), PPE-NCV1, ePPE (Zong, Y, Liu, Y., Xue, C. et al. An engineered prime editor with enhanced editing efficiency in plants. Nat Biotechnol 40, 1394-1402 (2022).) and ePPE-wtCas9 (replacing H840A-Cas9 in ePPE with wtCas9) constructs, using double pegRNA strategy to insert two recombinase recognition sites (RS) of Lox66 (34bp in length) and/or FRT1 (48bp in length) into the endogenous target site. The schematic diagram of the vector construction strategy is shown in Figure 1A.

Rice protoplasts were chosen as model cells. The above constructs were transformed into rice protoplasts by PEG transformation, and the efficiency of five prime editing system constructs using 5 pairs of pegRNAs to insert Lox66 or FRT1 at the endogenous sites of rice protoplasts was tested. The next-generation sequencing results are shown in Figure 1B .

The results show that when using the dual pegRNA strategy for site-specific insertion, the efficiency of using ePPE is the highest, which can increase the precision insertion efficiency by 10-50 times compared with PPE.

### 1.2. Screen of guide RNA

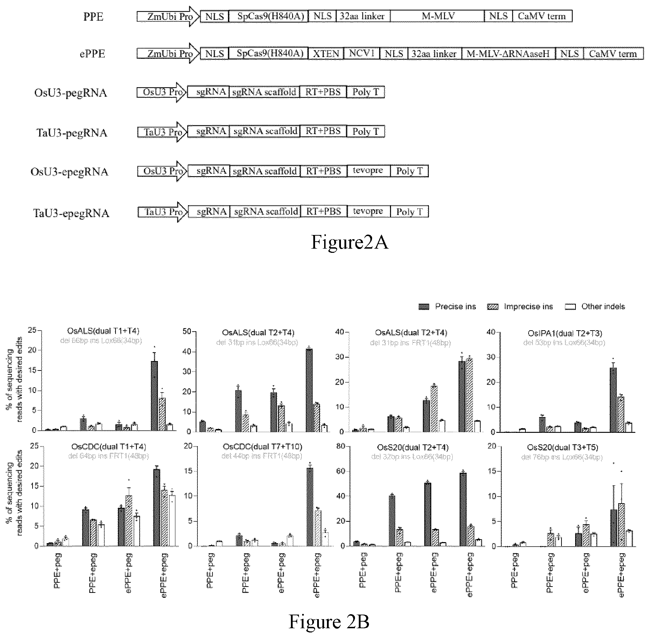

The editing efficiency of common pegRNA was further compared with the reported epegRNA containing tevoPre that can improve the efficiency of PE (Nelson, J.W., Randolph, PB., Shen, S.P. et al. Engineered pegRNAs improve prime editing efficiency. Nat Biotechnol 40, 402-410 (2022).) in different prime editing systems. Four combinations were tested, including PPE+pegRNA, ePPE+pegRNA, PPE+epegRNA, ePPE+epegRNA. Vector construction is shown in Figure 2A.

Rice protoplasts were also used to test the efficiency of the above four combinations using 8 pairs of pegRNA/epegRNA to insert RS at the site, and the next-generation sequencing results are shown in Figure 2B.

The results show that the combination of ePPE+epegRNA (hereinafter referred to as "dual-ePPE") had the highest efficiency of site-directed insertion mediated by the dual-pegRNA strategy, and the highest efficiency of some sites can reach more than 50%. Compared with the ordinary PPE+pegRNA combination, the maximum increase was more than 100 times, and it has higher efficiency in most of the inefficient targets. It is worth noting that the increase in the editing efficiency of editing tools for target sites is accompanied by an increase in the probability of inaccurate editing or insertion or deletion of other sites. Compared with other combinations, dual-ePPE has a significant improvement in the precise editing efficiency of the target site, while there is no significant change in the insertion or deletion efficiency of other sites.

### 1.3. Insertion by dual-ePPE

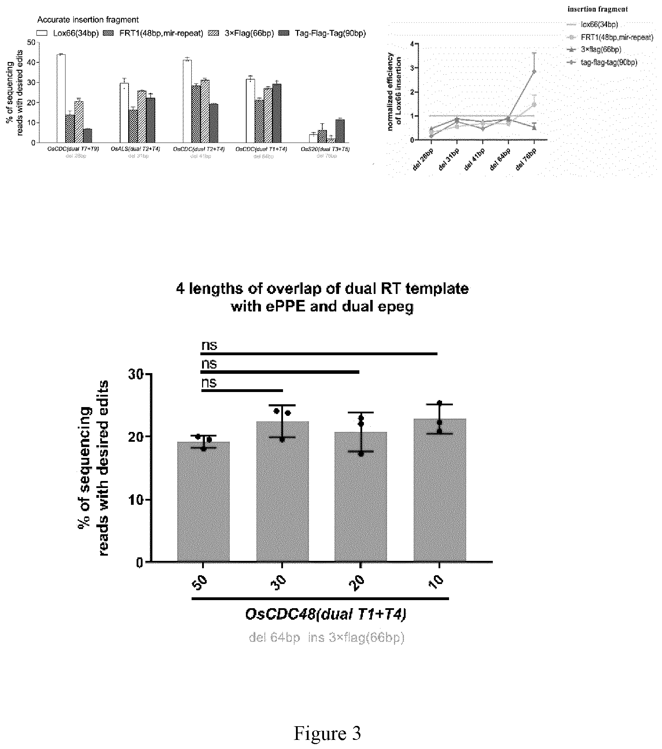

Further dual-ePPE was used to evaluate the relationship between the insertion length (30bp-100bp) and the distance between two pegRNAs (PAM distance 20bp-80bp), and the effect of the length of the overlap between the two RTs (10bp-50bp) on the insertion efficiency. Using the rice protoplast, the next generation sequencing results are shown in Figure 3.

The results show that there is no obvious linear relationship between the insertion length and the distance between pegRNAs. When the insertion length is greater than the distance between pegRNAs, the efficiency is higher, and the overlap length between the two RTs has a higher insertion efficiency between 10bp-50bp. The above results show that the ePPE+epegRNA system of the present invention can satisfy the efficient and site-specific insertion of tag sequences such as Flag and Tag.

### Example 2. Optimization of dual-ePPE system

In order to further verify and optimize the effect of the dual-ePPE system of the present invention under different usage environments, thereby obtaining an optimal technical solution, this example verifies and analyzes the possible improvements of each component of the dual-ePPE system.

### 2.1. CRISPR system effector proteins

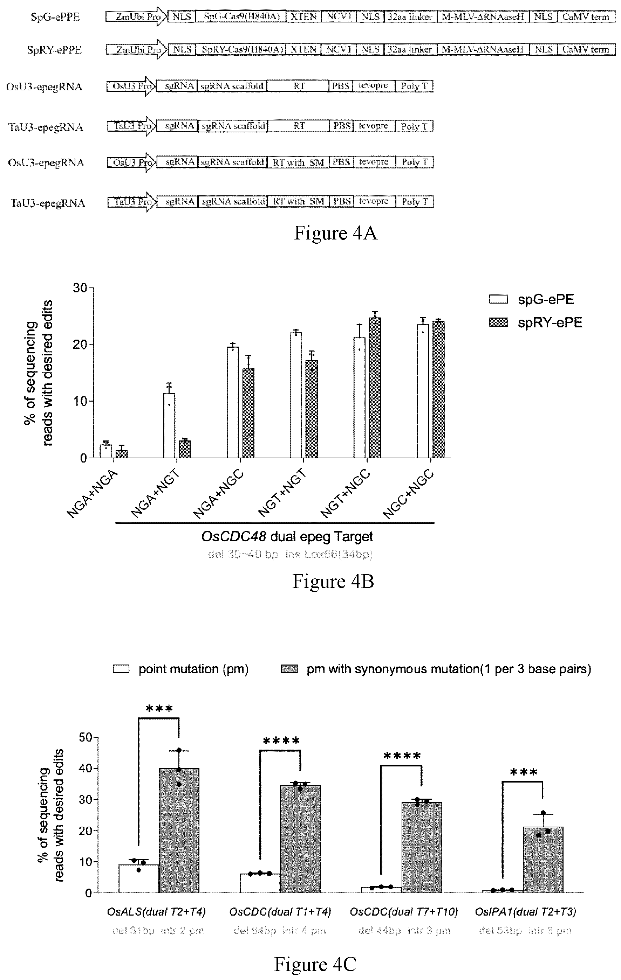

In this embodiment, SpG-Cas9 with NGN PAM and SpRY-Cas9 variants which are hardly restricted by PAM sequence (Christie KA, Guo JA, Silverstein RA, Doll RM, Mabuchi M, Stutzman HE, Lin J, Ma L , Walton RT, Pinello L, Robb GB, Kleinstiver BP. Precise DNA cleavage using CRISPR-SpRYgests. Nat Biotechnol. 2023 Mar;41(3):409-416.) were designed into dual-ePPE and evaluated its insertion efficiency, to expand the targeting range of dual-ePPE. Vector construction is shown in Figure 4A.

Rice protoplasts were used to test the efficiency of site-directed insertion under three PAM combinations of NGA, NGC and NGT. The results of next-generation sequencing are shown in Figure 4B.

The results showed that using SpG-ePPE and SpRY-ePPE to insert PAMs of NGA, NGC, and NGT also had high efficiency, which further verified that the dual-ePPE system can be applied to a variety of CRISPR system effector proteins and can effectively exert their functions. This result shows that the dual-ePPE of the present invention can effectively realize the insertion of RS sequences in plants.

### 2.2. Synonymous mutation in RT sequence

It has been reported that the introduction of synonymous mutation (synonymous mutation, SM) on the RT sequence can improve the efficiency of recombination editing (Xu, W., Yang, Y., Yang, B. et al. A design optimized prime editor with expanded scope and capability in plants. Nat. Plants 8, 45-52 (2022).). This example tested the editing efficiency of this system for SM processing at RT. Vector construction is shown in Figure 4A.

The efficiency of spiking using two RTs at four targets was tested using rice protoplasts. The next-generation sequencing results are shown in Figure 4C. The results show that the efficiency of point mutation can be greatly improved (4-20 times) when there is a uniform mismatch between the RT sequence and the genome sequence.

### 2.3. Promoters driving epegRNA expression

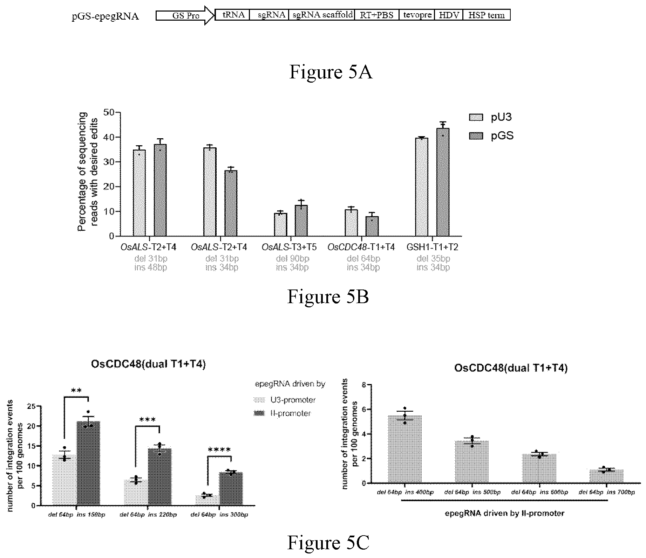

We further studied the efficiency of inserting longer fragments (150bp-300bp) using the above system, and tested the editing efficiency when the guide RNA was expressed from the U3 promoter and the composite type II promoter (pGS promoter). The pGS-epegRNA vector construction is shown in Figure 5A.

The efficiency of site-directed insertion of fragments of different lengths using U3 promoter and pGS promoter was compared in rice protoplasts. The ddPCR detection results are shown in Figure 5B and C. The results showed that there was no significant difference between the U3 promoter and the pGS promoter when inserting small fragments (Fig. 5B). When the insertion length reaches more than 150bp, the efficiency of pGS promoter to drive epegRNA is higher than that of U3 promoter. Using pGS promoter to express epegRNA can improve the insertion efficiency of large fragments by 2-5 times, and it can still be achieved when the length of the insertion fragment reaches 700bp Precise insertion.

### 2.4. Effect of MS2-MCP and altered temperature treatment on editing efficiency

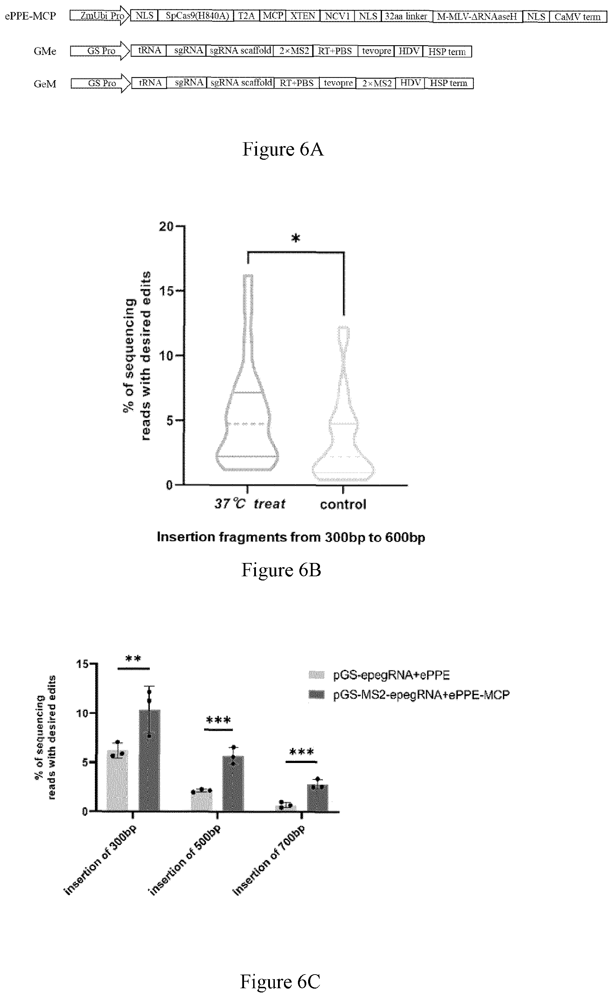

The MS2-MCP system for recruiting MLV was further used, and the temperature treatment at 37°C was used to further improve the efficiency of long fragment insertion. The vector construction is shown in Figure 6A.

The rice protoplasts were used to test the efficiency of the above two recruitment forms for large-segment fixed-point insertion, and also tested whether the method of 37 ° C treatment (TT, normal culture for 12h → 37° C for 12h → normal culture for 24h) could improve the efficiency. The ddPCR results are shown in Figure 6B.

The results showed that using 37°C temperature treatment can increase the insertion efficiency of large fragments by about 1.2-5 times (Figure 6B), and recruiting MLV using the MS2-MCP system can increase the insertion efficiency of large fragments by about 2-4 times (Figure 6C).

### Example 3. Using the PrimeROOT system to achieve non-double-strand break large fragment DNA insertion in plants

In this example, the combination of dual-ePPE and recombinase was used as Prime editing-mediated Recombination Of Opportune Targets (PrimeROOT for short), and its function of inserting DNA fragments in plants was verified .

### 3.1. Construction of fluorescent reporter system

In order to verify the DNA recombination ability of various recombinases in plant editing. First, the inventors constructed a fluorescent reporter system to characterize the DNA recombination efficiency of commonly used site-specific recombinases in rice protoplasts. This reporter system divides GFP into two domains, N-terminal (GFP-N) and C-terminal (GFP-C), which are encoded on two separate plasmids (Figure 7A), each carrying a recombinase site point, see Figure 7A for a schematic diagram of plasmid construction. After the expression and recombination of the recombinase, GFP-N and GFP-C are linked by an intron linker, so that GFP can be expressed in protoplasts. Furthermore, GFP fluorescence can be detected by fluorescence microscope observation and flow cytometry to characterize the recombinase activity in the protoplasts.

### 3.2. Construction of PrimeROOT for detection

The inventors constructed independent fluorescent reporter systems for 6 different tyrosine recombinases and 2 serine recombinases (all recombinases are codon-optimized and can be expressed in rice). GFP fluorescence microscope observation results (Figure 7B) and flow cytometry retrieval results (Figure 7C) show that the Cre and FLP recombinase system can produce the strongest fluorescence, and can be used as the best recombinase to verify and optimize the effectiveness of the technical solution of the present invention system.

In another set of parallel experiments, the inventors constructed a fluorescent reporter system for the Cre/Lox system of the tyrosine recombinase family, the FLP/FRT system, and the ΦC31 and Bxb1 recombinases of the serine family. The vector construction is shown in Figure 7D .

The above reporter system was transformed into rice protoplasts, and observed by fluorescence microscope and detected by flow cytometry, the results are shown in Figure 7E.

The results showed that Cre/Lox system and FLP/FRT system had stronger DNA integration ability. Therefore, it was combined with the above-mentioned site-specific insertion system, and all components (ePPE, two epegRNAs, recombinase, and the gene to be inserted with a recombination site) were transferred into rice cells through a "one-step method" to achieve large-scale gene expression at the gene level. The fragments are inserted at fixed points, as shown in Figure 7F. The "one-step" formulation containing dual-ePPE, recombinase, and the gene to be inserted with a recombination site is named PrimeROOT.v1, and is named according to whether the recombinase is the Cre/Lox system or the FLP/FRT system for PrimeROOT.v1-Cre and PrimeROOT.v1-FLP.

### 3.3. Capability verification of PrimeROOT.v1 large fragment insertion

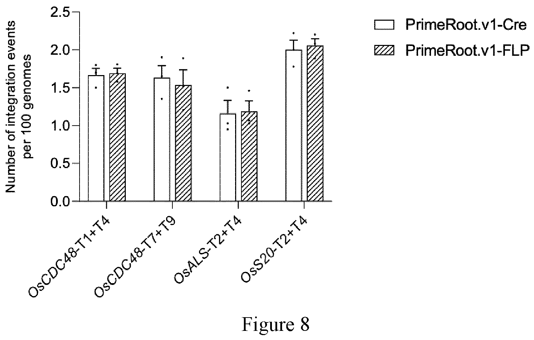

To verify the ability of PrimeROOT.v1 to insert large DNA molecules, the inventors tested the ability of PrimeROOT.v1-Cre and PrimeROOT.v1-FLP to GFP (720 kp) at four endogenous sites in rice protoplasts by ddPCR. Integration efficiency, the experimental results are shown in Figure 8. The results showed that both PrimeROOTs achieved precise, targeted large fragment insertions at all four sites.

### 3.4. Recombination system optimization

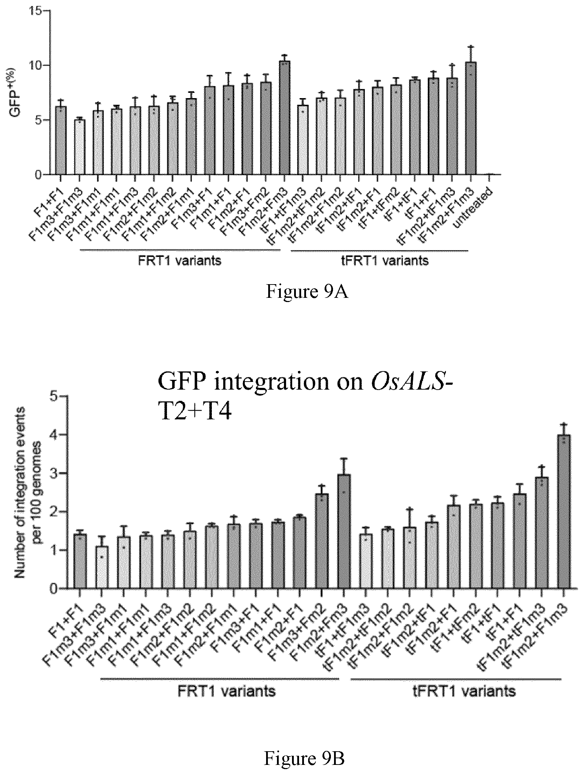

Due to the presence of short repeats in FRT1, some FRT1 mutants have been reported to promote the efficiency of FLP recombinase (Bruckner, R.C. & Cox, M.M. Specific Contacts between the Flp Protein of the Yeast 2-Micron Plasmid and Its Recombination Site. Journal of Biological Chemistry 261, 1798-1807 (1986).;Senecoff, J.F., Rossmeissl, P.J. & Cox, M.M. DNA recognition by the FLP recombinase of the yeast 2 mu plasmad. A mutational analysis of the FLP binding site. J Mol Biol 201 , 405-421 (1988).). In order to further optimize the editing system to obtain a more optimal technical solution. The inventors artificially designed multiple FRT1 mutants (F1m1, F1m2 and F1m3) and two truncated FRT1 (tFRT1) sequence mutants (tF1m2 and tF1m3). When using PrimeROOT for integration, the method of ddPCR evaluates the efficiency of one-step large fragment insertion on endogenous targets such as the fusion of the above recombinases and FRT variants, and uses one-step method to insert GFP into rice endogenous genes The protoplast cells are then made to glow. The results of ddPCR are shown in FIG. 9 , the combination of FRT1 mutants has higher mutation efficiency than the wild type.

### 3.5. PrimeROOT system optimization

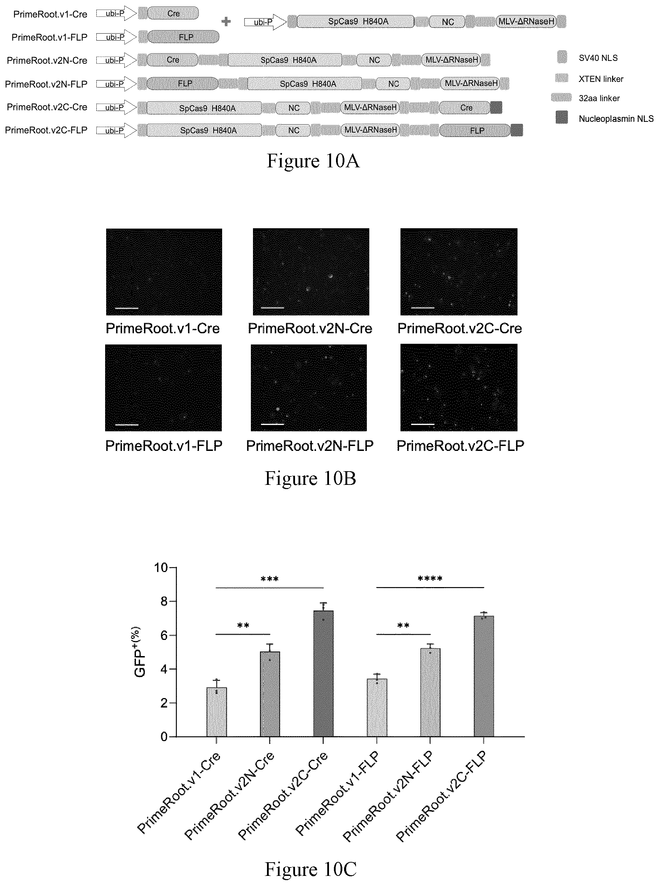

On the basis of PrimeROOT.v1, the inventor further optimized it to obtain a more optimal technical solution. In this technical solution, the inventor fused the ePPE of the PrimeROOT composite species with the recombinase, and created two structural solutions according to the different fusion sites. See Figure 10A for an example sequence:
Scheme 1 connects the recombinase to the N-terminal of the ePPE system through SV40 NLS and a flexible linker of 32 amino acids, named PrimeROOT.v2N; scheme 2 connects the recombinase to the C-terminal of the ePPE system through the same method, named PrimeROOT. v2C. Fluorescence microscope observation and flow cytometry results showed that PrimeROOT.v2N and PrimeROOT.v2C systems had higher GFP insertion efficiency at four endogenous sites than PrimeROOT.v1 (Figure 10).

### 3.6. PrimeROOT.v2 Large Fragment Insertion Ability Verification

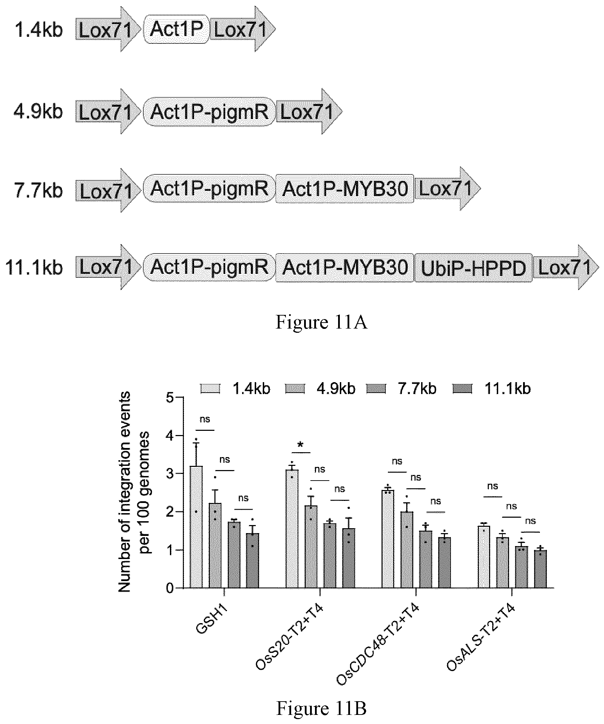

In order to verify the ability of PrimeROOT.v2 to insert large fragments of DNA molecules, the inventors constructed vector constructs containing any one or a combination of the three genes (pigmR, OsMYB30 and OsHPPD), and the lengths of the donors were 1.4 kb, 4.9 kb, 7.7 kb and 11.1 kb, the vector construction is shown in Figure 11A. The inventor detected the insertion efficiency of the four donors at the four endogenous sites by ddPCR, and found that with the gradual increase in the length of the donor, precise and targeted large fragment insertions were achieved, and the editing efficiency did not decrease significantly (FIG. 11B).

### Example 4. Using the PrimeROOT system to achieve non-double-strand break large fragment DNA insertion in maize species

In addition to rice protoplasts, the inventors also evaluated the editing efficiency of dual-ePPE and its PrimeROOT in maize protoplasts.

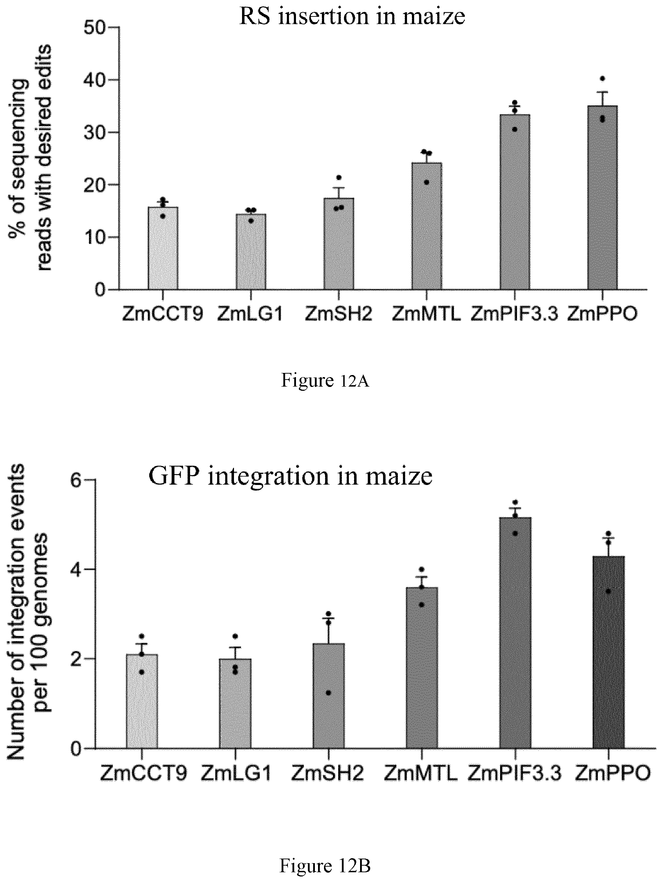

The inventors first tested the precise RS insertion editing efficiency of dual-ePPE at six endogenous gene loci in maize protoplasts, and the experimental results showed that it could achieve an editing efficiency as high as 40% (Figure 12A).

The inventors then tested the editing efficiency of PrimeROOT.v2C-Cre on GFP large fragment DNA, and the experimental results showed that it achieved a GFP sequence editing efficiency of up to 4% at the endogenous site (Figure 12B).

The experimental results are similar to the editing efficiency in rice, which indicates that the dual-ePPE of the present invention and the PrimeROOT system composed of it have broad and universal application prospects in plant synthetic biology and gene editing engineering, and can be inserted precisely The desired DNA sequence without introducing the donor backbone sequence.

### Example 5. Editing ability of PrimeROOT and CRISPR-mediated NHEJ system

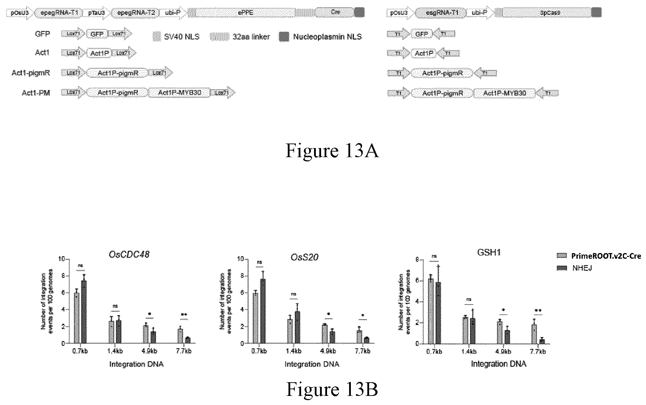

The CRISPR-mediated NHEJ system is currently reported as a system that can perform targeted large-segment insertions in plants (Li, J. et al. Gene replacements and insertions in rice by intron targeting using CRISPR-Cas9. Nature Plants 2 (2016 ).; Dong, O.X.O. et al. Marker-free carotenoid-enriched rice generated through targeted gene insertion using CRISPR-Cas9. Nature Communications 11 (2020).). In this example, taking PrimeROOT.v2C-Cre as an example, the PrimeROOT and CRISPR-mediated NHEJ systems were compared for GFP (720 bp), Act1 promoter (Act1P, 1.4 kb), Act1P-pigmR gene cassette (4.9 kb) and Act1P-pigmR-Act1P-OsMYB30 gene cassette (7.7 kb) for targeted insertion capability. The results showed that for the insertion of GFP and Act1P, the two had similar insertion efficiencies. But for longer donor insertions, the average efficiency of the PrimeROOT.v2C-Cre system is 2-4 times that of the NHEJ system (see Figure 13A for the construct schematic diagram and Figure 13B for the editing efficiency diagram).

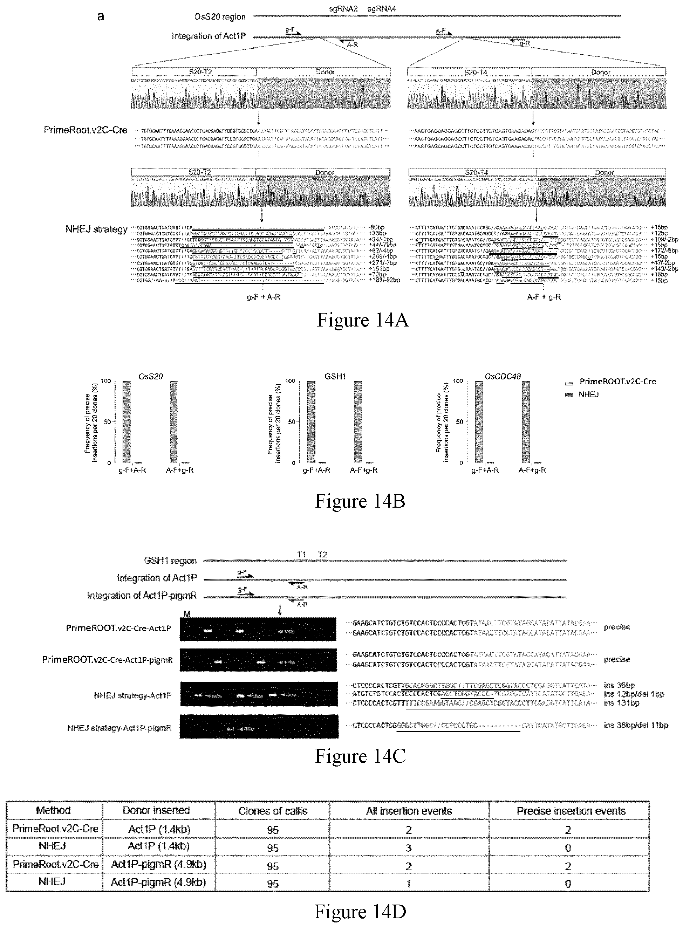

In terms of editing accuracy, the inventors observed that the Act1P event inserted by the PrimeROOT.v2C-Cre system showed clear Sanger sequencing results, but mixed peaks appeared in the results of insertion using NHEJ (Figure 14A, the underline indicates inaccurate insertion ). This indicates that the PrimeROOT system has superior editing accuracy compared to the traditional CRISPR-mediated NHEJ system.

The inventors then cloned the edited insertion events from protoplasts into bacteria and sequenced the junctions between the endogenous genome and the individual cloned inserts. When the inventors randomly selected 20 clones from a sample of Act1P insertions processed by PrimeROOT and NHEJ, the inventors found that all 20 insertions generated by PrimeROOT contained the exact inserted sequence as expected, whereas all 20 insertions generated by NHEJ Both contain random DNA base insertions and deletions/deletions at their junctions (Fig. 14A, B).

Next, the inventors inserted ActlP and Act1P-pigmR sequences into the genomic locus of rice calli using PrimeROOT and CRISPR-mediated NHEJ (Fig. 14C). Following transfer and induction of callus, the inventors analyzed 95 callus clones from each treatment to compare editing efficiency and precision. PrimeROOT generated 2 precise ActlP insertions and 2 precise Act1P-pigmR insertions, while NHEJ generated 3 imprecise Act1P insertions and 1 imprecise Act1P-pigmR insertion (the underline in Figure 14C represents the imprecise insertion, Figure 14D). These results demonstrate that PrimeROOT is an efficient editing tool for creating large, targeted and precise DNA insertions, in contrast to NHEJ systems that rely heavily on double-strand DNA breaks as intermediates.

### Example 6: Precise, targeted insertion of the actin promoter using the PrimeROOT tool

Many desirable agronomic traits are quantitative traits, depending on the up- or down-regulation of some specific genes, or on tissue-specific expression. In this example, the PrimeROOT system is used to precisely insert a favorable promoter upstream of the target gene, thereby realizing the application of the PrimeROOT tool in improving plant traits.

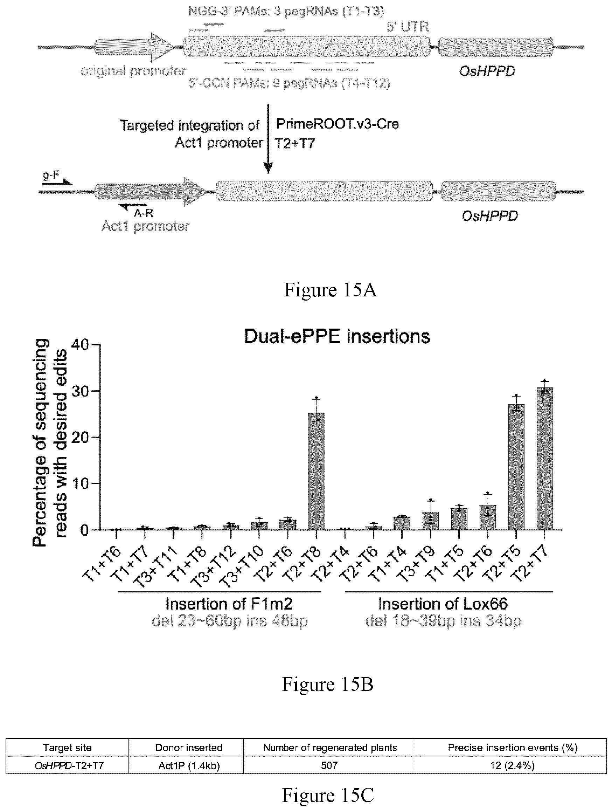

Specifically, the inventors used PrimeROOT.v2C-Cre to knock a strong promoter into the 5'UTR region of OsHPPD (Fig. 15A). In the first step, the inventors designed 16 pairs of pegRNAs in the 5'UTR, and compared their RS insertion editing efficiency in rice protoplasts, and determined that the RS insertion frequency of the best pegRNA pair was 30% (Figure 15B). Next, the inventors used PrimeROOT.v2C-Cre and the pegRNA pair to bombard rice Actin1 promoter (Act1P) particles into rice callus. The inventors identified edited plants by amplifying the junction between the genome and the inserted donor sequence, and assessed the insertion accuracy by Sanger sequencing. A total of 12 precise ActlP insertion events (2.4%) were detected in 507 regenerated rice plants (Fig. 15C). These results suggest that PrimeROOT can serve as an efficient genome insertion tool to introduce novel genetic regulatory elements into plant genomes for breeding.

### Example 7: Precise gene insertion in the GSH region

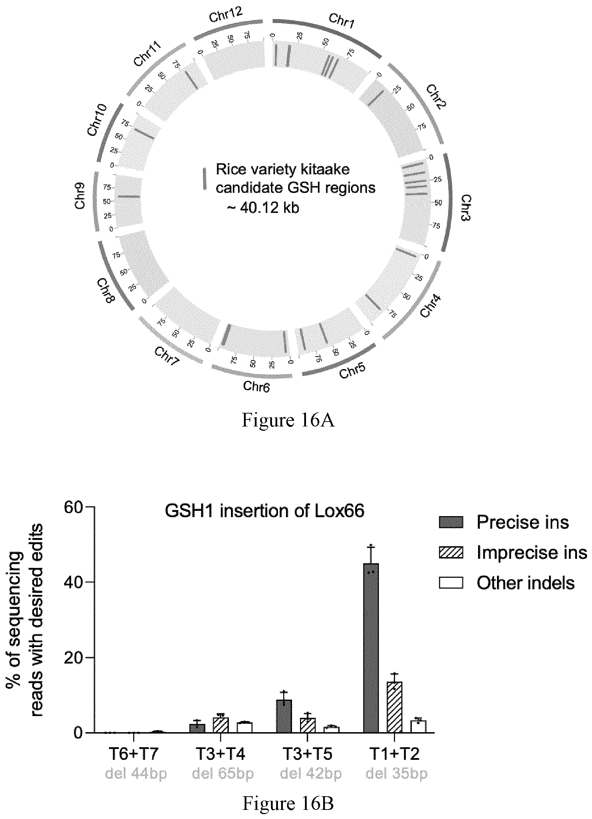

In order to ensure that the transgene can be safely inserted into the plant genome, the inventors predicted the genomic safe harbor (GSH) region of the entire Kitaake rice genome. Based on previous research methods on GSH (Aznauryan, E. et al. Discovery and validation of human genomic safe harbor sites for gene and cell therapies. Cell Rep Methods 2, 100154 (2022). ; Sadelain, M., Papapetrou, E.P. & Bushman, F.D. Safe harbors for the integration of new DNA in the human genome. Nat Rev Cancer 12, 51-58 (2011).), the inventors used a variety of algorithms to identify elements (such as gene coding regions, small RNA, miRNA, lncRNA, tRNA, promoter, enhancer, LTR, etc.) with a certain distance. In this way, the inventors generated a novel set of GSH regions consisting of 30 regions totaling 40 kb (Fig. 16A). All GSH regions of Kitaake are shown in Table 1. In addition, the inventors also identified 33 GSHs in the rice genome, and their mutually mapped GSH regions are shown in Table 2.

The inventors selected GSH1 (kitaake, Chr1:7660637-7661671) as a proof-of-concept region and designed 4 pairs of pegRNAs for RS insertion in this region (Table 3). When comparing the RS insertion efficiency using dual-ePPE in GSH1, the highest RS insertion efficiency was >40% (Fig. 16B). The inventors then detected the insertion of the 4.9 kb ActP1P-pigmRdonor cassette in the GSH1 region. Gel electrophoresis and Sanger sequencing results showed that 19 Act1-pigmR insertion events (2.6%) were identified in 744 regenerated plants. Importantly, all 19 ligations produced amplification products of the same size and were shown by sequencing to be the result of precise insertion events in which the end of the donor cassette was exactly as predicted.

### Example 8: Transfer method of PrimeROOT and donor

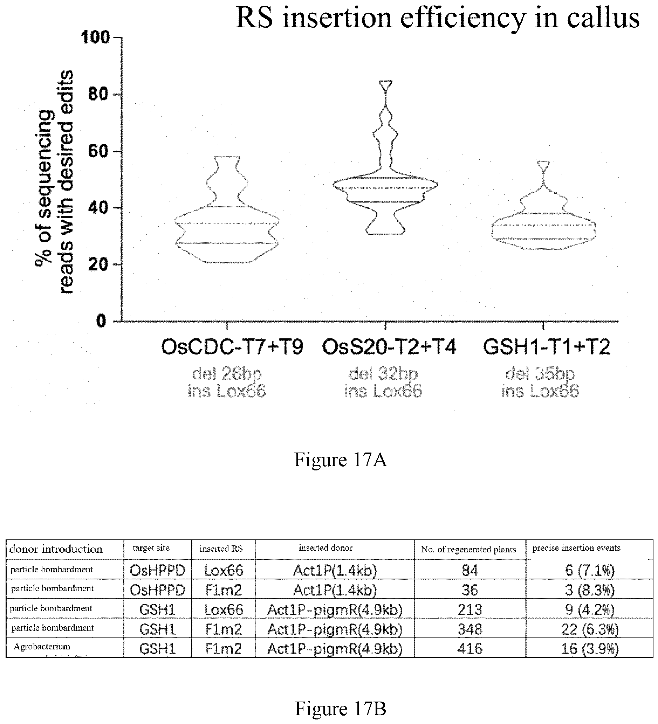

In order to test the insertion efficiency of the transformation method of PrimeROOT and donor components in the process of plant editing. The inventors used Lox66 and FRT mutant F1m2 as landing sites to test the recovery efficiency of PrimeROOT and donor components sequentially transformed into rice callus (the system of sequential transformation is called PrimeROOT.v3) on the overall edited plants. The inventors first evaluated dual-ePPE-mediated RS insertion in rice callus and achieved an editing efficiency as high as 84.7% (Fig. 17A). In the first round of transformation, the inventors transformed the PrimeROOT reagent (donor-free) into the calli by Agrobacterium, and after 1 month of hygromycin selection, the inventors enriched the callus containing the desired RS insertion. damage tissue. These calli were then used as substrates for a second round of transformation containing donor vectors delivered by particle bombardment or Agrobacterium. Following G418 selection and regeneration, the inventors examined the regenerated plants and measured the editing frequency of the desired insertion events (Figure 17B). The inventors found that the editing efficiencies of the Cre-Lox66 and FLP-F1m2 sites for the precise insertion of OsHPPD 5'UTR into ActlP were 7.1% and 8.3%, respectively, which were 3 times and 3.5 times higher than the efficiency of one-step transformation; When evaluating the editing efficiency of Act1P-pigmR precisely inserted into GSH1, the inventors obtained the efficiency of the Cre-Lox66 site as 4.2%, and the efficiency of the FLP-F1m2 site as 6.3%, respectively, which were 1.6 times and 2.4 times higher than when the integrated plant transformation was carried out. times. When the inventors delivered the donor by Agrobacterium transformation, the inventors obtained 3.9% efficiency of precise insertion events by Act1P-pigmR into the GSH1 site. These results demonstrate that PrimeROOT.v3 can be performed using different delivery methods and further improve the efficiency of precisely targeted gene insertion in plants.

### Example 9: Test of PrimeROOT large fragment insertion in human cells

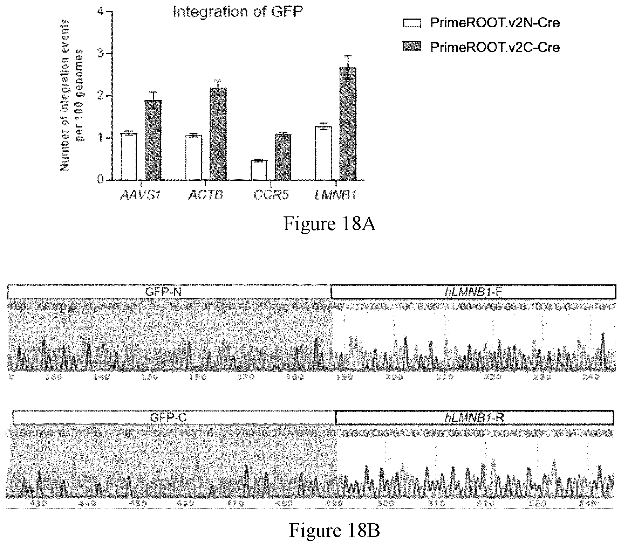

In order to test whether PrimeROOT works in human cells, the inventors first replaced the promoters of PrimeROOT.V2N-Cre and PrimeROOT.V2C-Cre with CMV promoters, which are commonly used in human cells. The inventors designed pegRNAs in the four regions of hAAVS1, hACTB, hCCR5, and hLMNB1 respectively, and constructed the pegRNAs on the expression vector of hU6, and then transformed the above plasmids and the donor plasmid containing GFP into the HEK293 cell line by plastid transformation In the process, DNA was extracted after 72 hours, followed by ddPCR to detect the efficiency (Figure 18A), and junction PCR was performed at the same time for first-generation sequencing detection, and it was found that the site-specific integration of GFP on the genome was completely accurate and predictable (Figure 18B) . This example shows that the PrimeROOT system has the function of precise targeted gene insertion in human cells.

**Table 1: Summary of GSH regions**

| **No.** | **Genome** | **chromosome** | **start site** | **stop site** | **length** |
|---|---|---|---|---|---|
| 1 | kitaake | Chr1 | 1492572 | 1494336 | 1764 |
| 2 | kitaake | Chr1 | 7272690 | 7273725 | 1035 |
| 3 | kitaake | Chr1 | 7275655 | 7277048 | 1393 |
| 4 | kitaake | Chr1 | 7660637 | 7661671 | 1034 |
| 5 | kitaake | Chr1 | 7664350 | 7665371 | 1021 |
| 6 | kitaake | Chr1 | 25529642 | 25530703 | 1061 |
| 7 | kitaake | Chr1 | 27199624 | 27201956 | 2332 |
| 8 | kitaake | Chr1 | 27202804 | 27203861 | 1057 |
| 9 | kitaake | Chr1 | 29416606 | 29418003 | 1397 |
| 10 | kitaake | Chr1 | 29705546 | 29706708 | 1162 |
| 11 | kitaake | Chr2 | 5940073 | 5941125 | 1052 |
| 12 | kitaake | Chr3 | 1631842 | 1633421 | 1579 |
| 13 | kitaake | Chr3 | 5736227 | 5738230 | 2003 |
| 14 | kitaake | Chr3 | 9480710 | 9481730 | 1020 |
| 15 | kitaake | Chr3 | 11887068 | 11888158 | 1090 |
| 16 | kitaake | Chr3 | 15273682 | 15274780 | 1098 |
| 17 | kitaake | Chr4 | 1830138 | 1832229 | 2091 |
| 18 | kitaake | Chr4 | 30059651 | 30060954 | 1303 |
| 19 | kitaake | Chr5 | 17071971 | 17074030 | 2059 |
| 20 | kitaake | Chr5 | 27239594 | 27241115 | 1521 |
| 21 | kitaake | Chr5 | 27241581 | 27242639 | 1058 |
| 22 | kitaake | Chr6 | 1704075 | 1705427 | 1352 |
| 23 | kitaake | Chr6 | 29383392 | 29384433 | 1041 |
| 24 | kitaake | Chr6 | 29388191 | 29389707 | 1516 |
| 25 | kitaake | Chr6 | 29891629 | 29893160 | 1531 |
| 26 | kitaake | Chr6 | 30055325 | 30056377 | 1052 |
| 27 | kitaake | Chr9 | 13406104 | 13407194 | 1090 |
| 28 | kitaake | Chr10 | 17574328 | 17575413 | 1085 |
| 29 | kitaake | Chr10 | 17576539 | 17577587 | 1048 |
| 30 | kitaake | Chr11 | 23793361 | 23794611 | 1250 |

**Table 2: Mutually mapped GSH regions of 33 rice genomes**

| Genome | Chr-start site-stop site |
|---|---|
| 2428 | Chr1:27105240-27107599 |
| Y58S | Chr1:26863329-26865688 |
| FH838 | Chr1:28113447-28115806 |
| DHX2 | Chr1:26800412-26802771 |
| WSSM | Chr1:27628585-27630944 |
| kitaake | Chr1:27199624-27201956 |
| ZH11 | Chr1:26863921-26866280 |
| LJ | Chr1:26712326-26714685 |
| Kosh | Chr1:26659192-26661551 |
| NamRoo | Chr1:27271081-27273440 |
| R498 | Chr1:27578972-27581331 |
| D62 | Chr1:27531324-27533683 |
| 9311 | Chr1:27292915-27295274 |
| R527 | Chr1:27699769-27702128 |
| G46 | Chr1:27195132-27197492 |
| Tumba | Chr1:27218330-27220689 |
| DG | Chr1:27165456-27167815 |
| CN1 | Chr1:27704214-27706574 |
| Y3551 | Chr1:27707251-27709610 |
| IR64 | Chr1:27422398-27424757 |
| FS32 | Chr1:27389099-27391458 |
| YX1 | Chr1:27638150-27640509 |
| S548 | Chr1:27422125-27424484 |
| zs97 | chr1:28028884-28031243 |
| MH63 | chr1:28396679-28399038 |
| KY131 | Chr1:26448216-26450575 |
| II32 | Chr1:27616163-27618522 |
| TM | Chr1:28051148-28053507 |
| G630 | Chr1:28040176-28042535 |
| G8 | Chr1:27349292-27351651 |
| MSU | Chr1:26477248-26479607 |
| J4155 | Chr1:27870434-27872793 |
| Lemont | Chr1:27364088-27365966 |

**Table 3 GSH1 validation designed pegRNA information**

| Target | prime sequence | RT template and PBS (Lower case letters)sequence |
|---|---|---|
| GSH1-T1 | GTCCACTCCCC ACTCGTGCA (SEQ ID NO:90) | ACCGTTCGTATAATGTATGCTATACGA AGTTATacgagtggg(SEQ ID NO:91) |
| GSH1-T2 | CTCTCCTACAA ACACAGGGT(SE Q ID NO:92) | CTTCGTATAGCATACATTATACGAACG GTActgtgtttgta(SEQ ID NO:93) |
| GSH1-T3 | TAGGAGAGAAA GAATATAGC(SE Q ID NO:94) | ACCGTTCGTATAATGTATGCTATACGA AGTTATatattctttctc(SEQ ID NO:95) |
| GSH1-T4 | AACCAAACGCA GACAGGACA(S EQ ID NO:96) | CTTCGTATAGCATACATTATACGAACG GTΛcctgtctgc(SEQ ID NO:97) |
| GSH1-T5 | TGAGAAGGAGC AAGCGAGAC(S EQ ID NO:98) | CTTCGTATAGCATACATTATACGAACG GTAtcgcttgct(SEQ ID NO:99) |
| GSH1-T6 | ATGCTAGATCC GTCCCTAGA(SE Q ID NO:100) | ACCGTTCGTATAATGTATGCTATACGA AGTTATatatagggacgga(SEQ ID NO:101) |
| GSH1-T7 | CCATGCATGAT GCATGGGAT(SE Q ID NO:102) | CTTCGTATAGCATACATTATACGAACG GTAccatgcatca(SEQ ID NO:103) |

Sequence information
>wildtype SpCas9 amino acid sequence (SEQ ID NO:1)






















>nCas9(H840A) amino acid sequence (SEQ ID NO:2)
MDKKYSIGLDIGTNSVGWAVITDEYKVPSKKFKVLGNTDRHSIKKNLIGALLFDSGETAEATRLKRT
ARRRYTRRKNRICYLQEIFSNEMAKVDDSFFHRLEESFLVEEDKKHERHPIFGNIVDEVAYHEKYPT
IYHLRKKLVDSTDKADLRLIYLALAHMIKFRGHFLIEGDLNPDNSDVDKLFIQLVQTYNQLFEENPI
NASGVDAKAILSARLSKSRRLENLIAQLPGEKKNGLFGNLIALSLGLTPNFKSNFDLAEDAKLQLSK
DTYDDDLDNLLAQIGDQYADLFLAAKNLSDAILLSDILRVNTEITKAPLSASMIKRYDEHHQDLTLL
KALVRQQLPEKYKEIFFDQSKNGYAGYIDGGASQEEFYKFIKPILEKMDGTEELLVKLNREDLLRK
QRTFDNGSIPHQIHLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNELTKVKYVTEGM
RKPAFLSGEQKKAIVDLLFKTNRKVTVKQLKEDYFKKIECFDSVEISGVEDRFNASLGTYHDLLKII
KDKDFLDNEENEDILEDIVLTLTLFEDREMIEERLKTYAHLFDDKVMKQLKRRRYTGWGRLSRKLI
NGIRDKQSGKTILDFLKSDGFANRNFMQLIHDDSLTFKEDIQKAQVSGQGDSLHEHIANLAGSPAIK
KGILQTVKVVDELVKVMGRHKPENIVIEMARENQTTQKGQKNSRERMKRIEEGIKELGSQILKEHP
VENTQLQNEKLYLYYLQNGRDMYVDQELDINRLSDYDVDAIVPQSFLKDDSIDNKVLTRSDKNRG
KSDNVPSEEVVKKMKNYWRQLLNAKLITQRKFDNLTKAERGGLSELDKAGFIKRQLVETRQITKH
VAQILDSRMNTKYDENDKLIREVKVITLKSKLVSDFRKDFQFYKVREINNYHHAHDAYLNAVVGT
ALIKKYPKLESEFVYGDYKVYDVRKMIAKSEQEIGKATAKYFFYSNIMNFFKTEITLANGEIRKRPL
IETNGETGEIVWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAKGYKEVKKDLI
IKLPKYSLFELENGRKRMLASAGELQKGNELALPSKYVNFLYLASHYEKLKGSPEDNEQKQLFVE
QHKHYLDEIIEQISEFSKRVILADANLDKVLSAYNKHRDKPIREQAENIIHLFTLTNLGAPAAFKYFD
TTIDRKRYTSTKEVLDATLIHQSITGLYETRIDLSQLGGD

>wildtype M-MLV-RT amino acid sequence (SEQ ID NO.3)
TLNIEDEYRLHETSKEPDVSLGSTWLSDFPQAWAETGGMGLAVRQAPLIIPLKATSTPVSIKQYPMS
QEARLGIKPHIQRLLDQGILVPCQSPWNTPLLPVKKPGTNDYRPVQDLREVNKRVEDIHPTVPNPY
NLLSGLPPSHQWYTVLDLKDAFFCLRLHPTSQPLFAFEWRDPEMGISGQLTWTRLPQGFKNSPTLF
NEALHRDLADFRIQHPDLILLQYVDDLLLAATSELDCQQGTRALLQTLGNLGYRASAKKAQICQK
QVKYLGYLLKEGQRWLTEARKETVMGQPTPKTPRQLREFLGKAGFCRLFIPGFAEMAAPLYPLTKP
GTLFNWGPDQQKAYQEIKQALLTAPALGLPDLTKPFELFVDEKQGYAKGVLTQKLGPWRRPVAYLS
KKLDPVAAGWPPCLRMVAAIAVLTKDAGKLTMGQPLVILAPHAVEALVKQPPDRWLSNARMTHY
QALLLDTDRVQFGPVVALNPATLLPLPEEGLQHNCLDILAEAHGTRPDLTDQPLPDADHTWYTDGS
SLLQEGQRKAGAAVTTETEVIWAKALPAGTSAQRAELIALTQALKMAEGKKLNVYTDSRYAFATA
HIHGEIYRRRGWLTSEGKEIKNKDEILALLKALFLPKRLSIIHCPGHQKGHSAEARGNRMADQAAR
KAAITETPDTSTLLIENSSP

>M-MLV-RT-connection(SEQ ID NO.4)
DQQKAYQEIKQALLTAPALGLPDLTKPFELFVDEKQGYAKGVLTQKLGPWRRPVAYLSKKLDPVAA
GWPPCLRMVAAIAVLTKDAGKLTMGQPLVILAPHAVEALVKQPPDRWLSNARMTHYQALLLDT

>RT-RNasc H (SEQ ID NO.5)
PDADHTWYTDGSSLLQEGQRKAGAAVTTETEVIWAKALPAGTSAQRAELIALTQALKMAEGKKL
NVYTDSRYAFATAHIHGEIYRRRGWLTSEGKEIKNKDEILALLKALFLPKRLSIIHCPGHQKGHSAEA
RGNRMADQAARKAAITETPDTSTLL

>NC(SEQ ID NO.6)
ATVVSGQKQDRQGGERRRSQLDRDQCAYCKEKGHWAKDCPKKPRGPRGPRPQTSLL

>PR(SEQ ID NO.7)
TLDDQGGQGQEPPPEPRITLKVGGQPVTFLVDTGAQHSVLTQNPGPLSDKSAWVQGATGGKRYRW
TTDRKVHLATGKVTHSFLHVPDCPYPLLGRDLLTKLKAQIHFEGSGAQVMGPMGQPLQVL

>IN(SEQ ID NO.8)
ENSSPYTSEHFHYTVTDIKDLTKLGAIYDKTKKYWVYQGKPVMPDQFTFELLDFLHQLTHLSFSK
MKALLERSHSPYYMLNRDRTLKNITETCKACAQVNASKSAVKQGTRVRGHRPGTHWEIDFTEIKP
GLYGYKYLLVFIDTFSGWIEAFPTKKETAKVVTKKLLEEIFPRFGMPQVLGTDNGPAFVSKVSQTVA
DLLGIDWKLHCAYRPQSSGQVERMNRTIKETLTKLTLATGSRDWVLLLPLALYRARNTPGPHGLTP
YEILYGAPPPLVNFPDPDMTRVTNSPSLQAHLQALYLVQHEVWRPLAAAYQEQLDRPVVPHPYRV
GDTVWVRRHQTKNLEPRWKGPYTVLLTTPTALKVDGIAAWIHAAHVKAADPGGGPSSRLTWRVQ
RSQNPLKIRLTREAP

> M-MLV-RT-F155Y(SEQ ID NO.9)
TLNIEDEYRLHETSKEPDVSLGSTWLSDFPQAWAETGGMGLAVRQAPLIIPLKATSTPVSIKQYPMS
QEARLGIKPHIQRLLDQGILVPCQSPWNTPLLPVKKPGTNDYRPVQDLREVNKRVEDIHPTVPNPY
NLLSGLPPSHQWYTVLDLKDAYFCLRLHPTSQPLFAFEWRDPEMGISGQLTWTRLPQGFKNSPTLF
NEALHRDLADFRIQHPDLILLQYVDDLLLAATSELDCQQGTRALLQTLGNLGYRASAKKAQICQK
QVKYLGYLLKEGQRWLTEARKETVMGQPTPKTPRQLREFLGKAGFCRLFIPGFAEMAAPLYPLTKP
GTLFNWGPDQQKAYQEIKQALLTAPALGLPDLTKPFELFVDEKQGYAKGVLTQKLGPWRRPVAYLS
KKLDPVAAGWPPCLRMVAAIAVLTKDAGKLTMGQPLVILAPHAVEALVKQPPDRWLSNARMTHY
QALLLDTDRVQFGPVVALNPATLLPLPEEGLQHNCLDILAEAHGTRPDLTDQPLPDADHTWYTDGS
SLLQEGQRKAGAAVTTETEVIWAKALPAGTSAQRAELIALTQALKMAEGKKLNVYTDSRYAFATA
HIHGEIYRRRGWLTSEGKEIKNKDEILALLKALFLPKRLSIIHCPGHQKGHSAEARGNRMADQAAR
KAAITETPDTSTLLIENSSP

> M-MLV-RT-F155V(SEQ ID NO.10)
TLNIEDEYRLHETSKEPDVSLGSTWLSDFPQAWAETGGMGLAVRQAPLIIPLKATSTPVSIKQYPMS
QEARLGIKPHIQRLLDQGILVPCQSPWNTPLLPVKKPGTNDYRPVQDLREVNKRVEDIHPTVPNPY
NLLSGLPPSHQWYTVLDLKDAVFCLRLHPTSQPLFAFEWRDPEMGISGQLTWTRLPQGFKNSPTLF
NEALHRDLADFRIQHPDLILLQYVDDLLLAATSELDCQQGTRALLQTLGNLGYRASAKKAQICQK
QVKYLGYLLKEGQRWLTEARKETVMGQPTPKTPRQLREFLGKAGFCRLFIPGFAEMAAPLYPLTKP
GTLFNWGPDQQKAYQEIKQALLTAPALGLPDLTKPFELFVDEKQGYAKGVLTQKLGPWRRPVAYLS
KKLDPVAAGWPPCLRMVAAIAVLTKDAGKLTMGQPLVILAPHAVEALVKQPPDRWLSNARMTHY
QALLLDTDRVQFGPVVALNPATLLPLPEEGLQHNCLDILAEAHGTRPDLTDQPLPDADHTWYTDGS
SLLQEGQRKAGAAVTTETEVIWAKALPAGTSAQRAELIALTQALKMAEGKKLNVYTDSRYAFATA
HIHGEIYRRRGWLTSEGKEIKNKDEILALLKALFLPKRLSIIHCPGHQKGHSAEARGNRMADQAAR
KAAITETPDTSTLLIENSSP

> M-MLV-RT-F156Y (SEQ ID NO.11)
TLNIEDEYRLHETSKEPDVSLGSTWLSDFPQAWAETGGMGLAVRQAPLIIPLKATSTPVSIKQYPMS
QEARLGIKPHIQRLLDQGILVPCQSPWNTPLLPVKKPGTNDYRPVQDLREVNKRVEDIHPTVPNPY
NLLSGLPPSHQWYTVLDLKDAFYCLRLHPTSQPLFAFEWRDPEMGISGQLTWTRLPQGFKNSPTLF
NEALHRDLADFRIQHPDLILLQYVDDLLLAATSELDCQQGTRALLQTLGNLGYRASAKKAQICQK
QVKYLGYLLKEGQRWLTEARKETVMGQPTPKTPRQLREFLGKAGFCRLFIPGFAEMAAPLYPLTKP
GTLFNWGPDQQKAYQEIKQALLTAPALGLPDLTKPFELFVDEKQGYAKGVLTQKLGPWRRPVAYLS
KKLDPVAAGWPPCLRMVAAIAVLTKDAGKLTMGQPLVILAPHAVEALVKQPPDRWLSNARMTHY
QALLLDTDRVQFGPVVALNPATLLPLPEEGLQHNCLDILAEAHGTRPDLTDQPLPDADHTWYTDGS

SLLQEGQRKAGAAVTTETEVIWAKALPAGTSAQRAELIALTQALKMAEGKKLNVYTDSRYAFATA
HIHGEIYRRRGWLTSEGKEIKNKDEILALLKALFLPKRLSIIHCPGHQKGHSAEARGNRMADQAAR
KAAITETPDTSTLLIENSSP

> M-MLV-RT-D524N (SEQ ID NO.12)
TLNIEDEYRLHETSKEPDVSLGSTWLSDFPQAWAETGGMGLAVRQAPLIIPLKATSTPVSIKQYPMS
QEARLGIKPHIQRLLDQGILVPCQSPWNTPLLPVKKPGTNDYRPVQDLREVNKRVEDIHPTVPNPY
NLLSGLPPSHQWYTVLDLKDAFFCLRLHPTSQPLFAFEWRDPEMGISGQLTWTRLPQGFKNSPTLF
NEALHRDLADFRIQHPDLILLQYVDDLLLAATSELDCQQGTRALLQTLGNLGYRASAKKAQICQK
QVKYLGYLLKEGQRWLTEARKETVMGQPTPKTPRQLREFLGKAGFCRLFIPGFAEMAAPLYPLTKP
GTLFNWGPDQQKAYQEIKQALLTAPALGLPDLTKPFELFVDEKQGYAKGVLTQKLGPWRRPVAYLS
KKLDPVAAGWPPCLRMVAAIAVLTKDAGKLTMGQPLVILAPHAVEALVKQPPDRWLSNARMTHY
QALLLDTDRVQFGPVVALNPATLLPLPEEGLQHNCLDILAEAHGTRPDLTDQPLPDADHTWYTNGS
SLLQEGQRKAGAAVTTETEVIWAKALPAGTSAQRAELIALTQALKMAEGKKLNVYTDSRYAFATA
HIHGEIYRRRGWLTSEGKEIKNKDEILALLKALFLPKRLSIIHCPGHQKGHSAEARGNRMADQAAR
KAAITETPDTSTLLIENSSP

> M-MLV-RT-N200C (SEQ ID NO.13)
TLNIEDEYRLHETSKEPDVSLGSTWLSDFPQAWAETGGMGLAVRQAPLIIPLKATSTPVSIKQYPMS
QEARLGIKPHIQRLLDQGILVPCQSPWNTPLLPVKKPGTNDYRPVQDLREVNKRVEDIHPTVPNPY
NLLSGLPPSHQWYTVLDLKDAYFCLRLHPTSQPLFAFEWRDPEMGISGQLTWTRLPQGFKNSPTLF
CEALHRDLADFRIQHPDLILLQYVDDLLLAATSELDCQQGTRALLQTLGNLGYRASAKKAQICQK
QVKYLGYLLKEGQRWLTEARKETVMGQPTPKTPRQLREFLGKAGFCRLFIPGFAEMAAPLYPLTKP
GTLFNWGPDQQKAYQEIKQALLTAPALGLPDLTKPFELFVDEKQGYAKGVLTQKLGPWRRPVAYLS
KKLDPVAAGWPPCLRMVAAIAVLTKDAGKLTMGQPLVILAPHAVEALVKQPPDRWLSNARMTHY
QALLLDTDRVQFGPVVALNPATLLPLPEEGLQHNCLDILAEAHGTRPDLTDQPLPDADHTWYTDGS
SLLQEGQRKAGAAVTTETEVIWAKALPAGTSAQRAELIALTQALKMAEGKKLNVYTDSRYAFATA
HIHGEIYRRRGWLTSEGKEIKNKDEILALLKALFLPKRLSIIHCPGHQKGHSAEARGNRMADQAAR
KAAITETPDTSTLLIENSSP

> M-MLV-RT-ΔRNase H (SEQ ID NO.14)
TLNIEDEYRLHETSKEPDVSLGSTWLSDFPQAWAETGGMGLAVRQAPLIIPLKATSTPVSIKQYPMS
QEARLGIKPHIQRLLDQGILVPCQSPWNTPLLPVKKPGTNDYRPVQDLREVNKRVEDIHPTVPNPY
NLLSGLPPSHQWYTVLDLKDAFFCLRLHPTSQPLFAFEWRDPEMGISGQLTWTRLPQGFKNSPTLF
NEALHRDLADFRIQHPDLILLQYVDDLLLAATSELDCQQGTRALLQTLGNLGYRASAKKAQICQK
QVKYLGYLLKEGQRWLTEARKETVMGQPTPKTPRQLREFLGKAGFCRLFIPGFAEMAAPLYPLTKP
GTLFNWGPDQQKAYQEIKQALLTAPALGLPDLTKPFELFVDEKQGYAKGVLTQKLGPWRRPVAYLS
KKLDPVAAGWPPCLRMVAAIAVLTKDAGKLTMGQPLVILAPHAVEALVKQPPDRWLSNARMTHY
QALLLDTDRVQFGPVVALNPATLLPLPEEGLQHNCLDILAEAHGTRPDLTDQPL

>M-MLV-RT-ΔRNase H-ΔConnection (SEQ ID NO.15)
TLNIEDEYRLHETSKEPDVSLGSTWLSDFPQAWAETGGMGLAVRQAPLIIPLKATSTPVSIKQYPMS
QEARLGIKPHIQRLLDQGILVPCQSPWNTPLLPVKKPGTNDYRPVQDLREVNKRVEDIHPTVPNPY
NLLSGLPPSHQWYTVLDLKDAFFCLRLHPTSQPLFAFEWRDPEMGISGQLTWTRLPQGFKNSPTLF
NEALHRDLADFRIQHPDLILLQYVDDLLLAATSELDCQQGTRALLQTLGNLGYRASAKKAQICQK
QVKYLGYLLKEGQRWLTEARKETVMGQPTPKTPRQLREFLGKAGFCRLFIPGFAEMAAPLYPLTKP
GTLFNWGP

>linker sequence (SEQ ID NO:16)
SGGSSGGSSGSETPGTSESATPESSGGSSGGS

>gRNA scaffold(SEQ ID NO:17)
guuuuagagcuagaaauagcaaguuaaaauaaggcuaguccguuaucaacuugaaaaaguggcaccgagucggugc

>PPE(SEQ ID NO:18)
MPKKKRKVDKKYSIGLDIGTNSVGWAVITDEYKVPSKKFKVLGNTDRHSIKKNLIGALLFDSGETA
EATRLKRTARRRYTRRKNRICYLQEIFSNEMAKVDDSFFHRLEESFLVEEDKKHERHPIFGNIVDEV
AYHEKYPTIYHLRKKLVDSTDKADLRLIYLALAHMIKFRGHFLIEGDLNPDNSDVDKLFIQLVQTY
NQLFEENPINASGVDAKAILSARLSKSRRLENLIAQLPGEKKNGLFGNLIALSLGLTPNFKSNFDLAE
DAKLQLSKDTYDDDLDNLLAQIGDQYADLFLAAKNLSDAILLSDILRVNTEITKAPLSASMIKRYD

EHHQDLTLLKALVRQQLPEKYKEIFFDQSKNGYAGYIDGGASQEEFYKFIKPILEKMDGTEELLVKL
NREDLLRKQRTFDNGSIPHQIHLGELHAILRRQEDFYPFLKDNREKIEKILTFRIPYYVGPLARGNSR
FAWMTRKSEETITPWNFEEVVDKGASAQSFIERMTNFDKNLPNEKVLPKHSLLYEYFTVYNELTKV
KYVTEGMRKPAFLSGEQKKAIVDLLFKTNRKVTVKQLKEDYFKKIECFDSVEISGVEDRFNASLGT
YHDLLKIIKDKDFLDNEENEDILEDIVLTLTLFEDREMIEERLKTYAHLFDDKVMKQLKRRRYTGW
GRLSRKLINGIRDKQSGKTILDFLKSDGFANRNFMQLIHDDSLTFKEDIQKAQVSGQGDSLHEHIAN
LAGSPAIKKGILQTVKVVDELVKVMGRHKPENIVIEMARENQTTQKGQKNSRERMKRIEEGIKELG
SQILKEHPVENTQLQNEKLYLYYLQNGRDMYVDQELDINRLSDYDVDAIVPQSFLKDDSIDNKVLT
RSDKNRGKSDNVPSEEVVKKMKNYWRQLLNAKLITQRKFDNLTKAERGGLSELDKAGFIKRQLV
ETRQITKHVAQILDSRMNTKYDENDKLIREVKVITLKSKLVSDFRKDFQFYKVREINNYHHAHDAY
LNAVVGTALIKKYPKLESEFVYGDYKVYDVRKMIAKSEQEIGKATAKYFFYSNIMNFFKTEITLAN
GEIRKRPLIETNGETGEIVWDKGRDFATVRKVLSMPQVNIVKKTEVQTGGFSKESILPKRNSDKLIA
RKKDWDPKKYGGFDSPTVAYSVLVVAKVEKGKSKKLKSVKELLGITIMERSSFEKNPIDFLEAKGY
KEVKKDLIIKLPKYSLFELENGRKRMLASAGELQKGNELALPSKYVNFLYLASHYEKLKGSPEDNE
QKQLFVEQHKHYLDEIIEQISEFSKRVILADANLDKVLSAYNKHRDKPIREQAENIIHLFTLTNLGAP
AAFKYFDTTIDRKRYTSTKEVLDATLIHQSITGLYETRIDLSQLGGDEFPKKKRKVELSGGSSGGSSG
SETPGTSESATPESSGGSSGGSRPTLNIEDEYRLHETSKEPDVSLGSTWLSDFPQAWAETGGMGLAV
RQAPLIIPLKATSTPVSIKQYPMSQEARLGIKPHIQRLLDQGILVPCQSPWNTPLLPVKKPGTNDYRP
VQDLREVNKRVEDIHPTVPNPYNLLSGLPPSHQWYTVLDLKDAFFCLRLHPTSQPLFAFEWRDPEM
GISGQLTWTRLPQGFKNSPTLFNEALHRDLADFRIQHPDLILLQYVDDLLLAATSELDCQQGTRALL
QTLGNLGYRASAKKAQICQKQVKYLGYLLKEGQRWLTEARKETVMGQPTPKTPRQLREFLGKAG
FCRLFIPGFAEMAAPLYPLTKPGTLFNWGPDQQKAYQEIKQALLTAPALGLPDLTKPFELFVDEKQG
YAKGVLTQKLGPWRRPVAYLSKKLDPVAAGWPPCLRMVAAIAVLTKDAGKLTMGQPLVILAPHAV
EALVKQPPDRWLSNARMTHYQALLLDTDRVQFGPVVALNPATLLPLPEEGLQHNCLDILAEAHGT
RPDLTDQPLPDADHTWYTDGSSLLQEGQRKAGAAVTTETEVIWAKALPAGTSAQRAELIALTQAL
KMAEGKKLNVYTDSRYAFATAHIHGEIYRRRGWLTSEGKEIKNKDEILALLKALFLPKRLSIIHCPG
HQKGHSAEARGNRMADQAARKAAITETPDTSTLLIENSSPSGGSPKKKRKV

>ePPE(SEQ ID NO:19)
MPKKKRKVDKKYSIGLDIGTNSVGWAVITDEYKVPSKKFKVLGNTDRHSIKKNLIGALLFDSGETA
EATRLKRTARRRYTRRKNRICYLQEIFSNEMAKVDDSFFHRLEESFLVEEDKKHERHPIFGNIVDEV
AYHEKYPTIYHLRKKLVDSTDKADLRLIYLALAHMIKFRGHFLIEGDLNPDNSDVDKLFIQLVQTY
NQLFEENPINASGVDAKAILSARLSKSRRLENLIAQLPGEKKNGLFGNLIALSLGLTPNFKSNFDLAE
DAKLQLSKDTYDDDLDNLLAQIGDQYADLFLAAKNLSDAILLSDILRVNTEITKAPLSASMIKRYD
EHHQDLTLLKALVRQQLPEKYKEIFFDQSKNGYAGYIDGGASQEEFYKFIKPILEKMDGTEELLVKL
NREDLLRKQRTFDNGSIPHQIHLGELHAILRRQEDFYPFLKDNREKIEKILTFRIPYYVGPLARGNSR
FAWMTRKSEETITPWNFEEVVDKGASAQSFIERMTNFDKNLPNEKVLPKHSLLYEYFTVYNELTKV
KYVTEGMRKPAFLSGEQKKAIVDLLFKTNRKVTVKQLKEDYFKKIECFDSVEISGVEDRFNASLGT
YHDLLKIIKDKDFLDNEENEDILEDIVLTLTLFEDREMIEERLKTYAHLFDDKVMKQLKRRRYTGW
GRLSRKLINGIRDKQSGKTILDFLKSDGFANRNFMQLIHDDSLTFKEDIQKAQVSGQGDSLHEHIAN
LAGSPAIKKGILQTVKVVDELVKVMGRHKPENIVIEMARENQTTQKGQKNSRERMKRIEEGIKELG
SQILKEHPVENTQLQNEKLYLYYLQNGRDMYVDQELDINRLSDYDVDAIVPQSFLKDDSIDNKVLT
RSDKNRGKSDNVPSEEVVKKMKNYWRQLLNAKLITQRKFDNLTKAERGGLSELDKAGFIKRQLV
ETRQITKHVAQILDSRMNTKYDENDKLIREVKVITLKSKLVSDFRKDFQFYKVREINNYHHAHDAY
LNAVVGTALIKKYPKLESEFVYGDYKVYDVRKMIAKSEQEIGKATAKYFFYSNIMNFFKTEITLAN
GEIRKRPLIETNGETGEIVWDKGRDFATVRKVLSMPQVNIVKKTEVQTGGFSKESILPKRNSDKLIA
RKKDWDPKKYGGFDSPTVAYSVLVVAKVEKGKSKKLKSVKELLGITIMERSSFEKNPIDFLEAKGY
KEVKKDLIIKLPKYSLFELENGRKRMLASAGELQKGNELALPSKYVNFLYLASHYEKLKGSPEDNE
QKQLFVEQHKHYLDEIIEQISEFSKRVILADANLDKVLSAYNKHRDKPIREQAENIIHLFTLTNLGAP
AAFKYFDTTIDRKRYTSTKEVLDATLIHQSITGLYETRIDLSQLGGDEFSGSETPGTSESATPESATVV
SGQKQDRQGGERRRSQLDRDQCAYCKEKGHWAKDCPKKPRGPRGPRPQTSLLPKKKRKVELSGG
SSGGSSGSETPGTSESATPESSGGSSGGSRPTLNIEDEYRLHETSKEPDVSLGSTWLSDFPQAWAETG
GMGLAVRQAPLIIPLKATSTPVSIKQYPMSQEARLGIKPHIQRLLDQGILVPCQSPWNTPLLPVKKPG
TNDYRPVQDLREVNKRVEDIHPTVPNPYNLLSGLPPSHQWYTVLDLKDAFFCLRLHPTSQPLFAFE
WRDPEMGISGQLTWTRLPQGFKNSPTLFNEALHRDLADFRIQHPDLILLQYVDDLLLAATSELDCQ
QGTRALLQTLGNLGYRASAKKAQICQKQVKYLGYLLKEGQRWLTEARKETVMGQPTPKTPRQLR
EFLGKAGFCRLFIPGFAEMAAPLYPLTKPGTLFNWGPDQQKAYQEIKQALLTAPALGLPDLTKPFEL
FVDEKQGYAKGVLTQKLGPWRRPVAYLSKKLDPVAAGWPPCLRMVAAIAVLTKDAGKLTMGQPL
VILAPHAVEALVKQPPDRWLSNARMTHYQALLLDTDRVQFGPVVALNPATLLPLPEEGLQHNCLDI
LAEAHGTRPDLTDQPLSGGSPKKKRKV

>tevopre (SEQ ID NO:20)
CGCGGTTCTATCTAGTTACGCGTTAAACCAACTAGAA

>pGS (SEQ ID NO:21)
Atggagtcaaagattcaaatagaggacctaacagaactcgccgtaaagactggcgaacagttcatacagagtctcttacgactcaatgacaagaagaaaat
cttcgtcaacatggtggagcacgacacacttgtctactccaaaaatatcaaagatacagtctcagaagaccaaagggcaattgagacttttcaacaaagggta
atatccggaaacctcctcggattccattgcccagctatctgtcactttattgtgaagatagtggaaaaggaaggtggctcctacaaatgccatcattgcgataaa
ggaaaggccatcgttgaagatgcctctgccgacagtggtcccaaagatggacccccacccacgaggagcatcgtggaaaaagaagacgttccaaccac
gtcttcaaagcaagtggattgatgtgattggcagacatactgtcccacaaatgaagatggaatctgtaaaagaaaacgcgtgaaataatgcgtctgacaaag
gttaggtcggctgcctttaatcaataccaaagtggtccctaccacgatggaaaaactgtgcagtcggtttggctttttctgacgaacaaataagattcgtggcc
gacaggtgggggtccaccatgtgaaggcatcttcagactccaataatggagcaatgacgtaagggcttacgaaataagtaagggtagtttgggaaatgtcc
actcacccgtcagtctataaatacttagcccctccctcattgttaagggagcaaaatctcagagagatagtcctagagagagaaagagagcaagtagcctag
aagtagtcaaggcggcgaagtattcaggcacgtggccaggaagaagaaaagccaagacgacgaaaacaggtaagagctaagcatctagataagttgaa
aacaatcttcaaaagtcccacatcgcttagataagaaaacgaagctgagtttatatacagctagagtcgaagtagtgatt

> ΦC31 (SEQ ID NO:22)
MDTYAGAYDRQSRERENSSAASPATQRSANEDKAADLQREVERDGGRFRFVGHFSEAPGTSAFGT
AERPEFERILNECRAGRLNMIIVYDVSRFSRLKVMDAIPIVSELLALGVTIVSTQEGVFRQGNVMDL
IHLIMRLDASHKESSLKSAKILDTKNLQRELGGYVGGKAPYGFELVSETKEITRNGRMVNVVINKL
AHSTTPLTGPFEFEPDVIRWWWREIKTHKHLPFKPGSQAAIHPGSITGLCKRMDADAVPTRGETIGK
KTASSAWDPATVMRILRDPRIAGFAAEVIYKKKPDGTPTTKIEGYRIQRDPITLRPVELDCGPIIEPAE
WYELQAWLDGRGRGKGLSRGQAILSAMDKLYCECGAVMTSKRGEESIKDSYRCRRRKVVDPSAP
GQHEGTCNVSMAALDKFVAERIFNKIRHAEGDEETLALLWEAARRFGKLTEAPEKSGERANLVAE
RADALNALEELYEDRAAGAYDGPVGRKHFRKQQAALTLRQQGAEERLAELEAAEAPKLPLDQWF
PEDADADPTGPKSWWGRASVDDKRVFVGLFVDKIVVTKSTTGRGQGTPIEKRASITWAKPPTDDD
EDDAQDGTEDVAA

> Bxb1(SEQ ID NO:23)
MRALVVIRLSRVTDATTSPERQLESCQQLCAQRGWDVVGVAEDLDVSGAVDPFDRKRRPNLARWL
AFEEQPFDVIVAYRVDRLTRSIRHLQQLVHWAEDHKKLVVSATEAHFDTTTPFAAVVIALMGTVAQ
MELEAIKERNRSAAHFNIRAGKYRGSLPPWGYLPTRVDGEWRLVPDPVQRERILEVYHRVVDNHE
PLHLVAHDLNRRGVLSPKDYFAQLQGREPQGREWSATALKRSMISEAMLGYATLNGKTVRDDDGA
PLVRAEPILTREQLEALRAELVKTSRAKPAVSTPSLLLRVLFCAVCGEPAYKFAGGGRKHPRYRCRS
MGFPKHCGNGTVAMAEWDAFCEEQVLDLLGDAERLEKVWVAGSDSAVELAEVNAELVDLTSLIG
SPAYRAGSPQREALDARIAALAARQEELEGLEARPSGWEWRETGQRFGDWWREQDTAAKNTWL
RSMNVRLTFDVRGGLTRTIDFGDLQEYEQHLRLGSVVERLHTGMSEF

>Cre (SEQ ID NO:24)
SNLLTVHQNLPALPVDATSDEVRKNLMDMFRDRQAFSEHTWKMLLSVCRSWAAWCKLNNRKWF
PAEPEDVRDYLLYLQARGLAVKTIQQHLGQLNMLHRRSGLPRPSDSNAVSLVMRRIRKENVDAGE
RAKQALAFERTDFDQVRSLMENSDRCQDIRNLAFLGIAYNTLLRIAEIARIRVKDISRTDGGRMLIHI
GRTKTLVSTAGVEKALSLGVTKLVERWISVSGVADDPNNYLFCRVRKNGVAAPSATSQLSTRALEG
IFEATHRLIYGAKDDSGQRYLAWSGHSARVGAARDMARAGVSIPEIMQAGGWTNVNIVMNYIRNL
DSETGAMVRLLEDGD

> FLP (SEQ ID NO:25)
MPQFDILCKTPPKVLVRQFVERFERPSGEKIALCAAELTYLCWMITHNGTAIKRATFMSYNTIISNSL
SFDIVNKSLQFKYKTQKATILEASLKKLIPAWEFTIIPYYGQKHQSDITDIVSSLQLQFESSEEADKGN
SHSKKMLKALLSEGESIWEITEKILNSFEYTSRFTKTKTLYQFLFLATFINCGRFSDIKNVDPKSFKLV
QNKYLGVIIQCLVTETKTSVSRHIYFFSARGRIDPLVYLDEFLRNSEPVLKRVNRTGNSSSNKQEYQL
LKDNLVRSYNKALKKNAPYSIFAIKNGPKSHIGRHLMTSFLSMKGLTELTNVVGNWSDKRASAVAR
TTYTHQITAIPDHYFALVSRYYAYDPISKEMIALKDETNPIEEWQHIEQLKGSAEGSIRYPAWNGIISQ
EVLDYLSSYINRRI

>LoxP (SEQ ID NO:26)
ATAACTTCGTATAGCATACATTATACGAAGTTAT

Lox66 (SEQ ID NO:27)
ATAACTTCGTATAGCATACATTATACGAACGGTA

Lox71 (SEQ ID NO:28)
taccgTTCGTATAGCATACATTATACGAAGTTAT

Lox2272 (SEQ ID NO:29)
Ataacttcgtataggatactttatacgaagttat

FRT1 (SEQ ID NO:30)
GAAGTTCCTATTCCGAAGTTCCTATTCTCTAGAAAGTATAGGAACTTC

FRT6 (SEQ ID NO:31)
Gaagttcctattccgaagttcctattcttcaaaaagtataggaacttc

FRT1m1 (SEQ ID NO:32)
GgAGgTCtTATTtCGAAGTTCCTATTCTCTAGAAAGTATAGGAACTTC

FRT1m2 (SEQ ID NO:33)
GAAGTTCCTATTCCGgAGgTCtTATTtTCTAGAAAGTATAGGAACTTC

FRT1m3 (SEQ ID NO:34)
GgAGgTCtTATTtCGAAGTTCCTATTCTCTAGAAAGTATAaGAcCTcC

mFRT1 (SEQ ID NO:35)
GAAGTTCCTATTCTCTAGAAAGTATAGGAACTTC

mFRT1m1 (SEQ ID NO:36)
GgAGgTCtTATTtTCTAGAAAGTATAGGAACTTC

mFRT1m2 (SEQ ID NO:37)
GAAGTTCCTATTCTCTAGAAAGTATAaGAcCTcC

aTTP (SEQ ID NO:38)
gtagtgccccaactggggtaacctttgagttctctcagttgggggcgtag

aTTB (SEQ ID NO:39)
cggtgcgggtgccagggcgtgcccttgggctccccgggcgcgtactccac

aGTP (SEQ ID NO:40)
GGTTTGTCTGGTCAACCACCGCGGTCTCAGTGGTGTACGGTACAAACC

aGTB (SEQ ID NO:41)
GGCCGGCTTGTCGACGACGGCGGTCTCCGTCGTCAGGATCATCCGG

SpG-nCas9 (SEQ ID NO:42)
KDKKYSIGLDIGTNSVGWAVITDEYKVPSKKFKVLGNTDRHSIKKNLIGALLFDSGETAEATRLKRT
ARRRYTRRKNRICYLQEIFSNEMAKVDDSFFHRLEESFLVEEDKKHERHPIFGNIVDEVAYHEKYPT
IYHLRKKLVDSTDKADLRLIYLALAHMIKFRGHFLIEGDLNPDNSDVDKLFIQLVQTYNQLFEENPI
NASGVDAKAILSARLSKSRRLENLIAQLPGEKKNGLFGNLIALSLGLTPNFKSNFDLAEDAKLQLSK
DTYDDDLDNLLAQIGDQYADLFLAAKNLSDAILLSDILRVNTEITKAPLSASMIKRYDEHHQDLTLL
KALVRQQLPEKYKEIFFDQSKNGYAGYIDGGASQEEFYKFIKPILEKMDGTEELLVKLNREDLLRK
QRTFDNGSIPHQIHLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNELTKVKYVTEGM
RKPAFLSGEQKKAIVDLLFKTNRKVTVKQLKEDYFKKIECFDSVEISGVEDRFNASLGTYHDLLKII
KDKDFLDNEENEDILEDIVLTLTLFEDREMIEERLKTYAHLFDDKVMKQLKRRRYTGWGRLSRKLI
NGIRDKQSGKTILDFLKSDGFANRNFMQLIHDDSLTFKEDIQKAQVSGQGDSLHEHIANLAGSPAIK
KGILQTVKVVDELVKVMGRHKPENIVIEMARENQTTQKGQKNSRERMKRIEEGIKELGSQILKEHP
VENTQLQNEKLYLYYLQNGRDMYVDQELDINRLSDYDVDAIVPQSFLKDDSIDNKVLTRSDKNRG
KSDNVPSEEVVKKMKNYWRQLLNAKLITQRKFDNLTKAERGGLSELDKAGFIKRQLVETRQITKH
VAQILDSRMNTKYDENDKLIREVKVITLKSKLVSDFRKDFQFYKVREINNYHHAHDAYLNAVVGT
ALIKKYPKLESEFVYGDYKVYDVRKMIAKSEQEIGKATAKYFFYSNIMNFFKTEITLANGEIRKRPL

IETNGETGEIVWDKGRDFATVRKVLSMPQVNIVKKTEVQTGGFSKESILPKRNSDKLIARKKDWDP
KKYGGFLWPTVAYSVLVVAKVEKGKSKKLKSVKELLGITIMERSSFEKNPIDFLEAKGYKEVKKDL
IIKLPKYSLFELENGRKRMLASAKQLQKGNELALPSKYVNFLYLASHYEKLKGSPEDNEQKQLFVE
QHKHYLDEIIEQISEFSKRVILADANLDKVLSAYNKHRDKPIREQAENIIHLFTLTNLGAPAAFKYFD
TTIDRKQYRSTKEVLDATLIHQSITGLYETRIDLSQLGGD

SpRY-nCas9：(SEQ ID NO:43)
DKKYSIGLDIGTNSVGWAVITDEYKVPSKKFKVLGNTDRHSIKKNLIGALLFDSGETAERTRLKRTA
RRRYTRRKNRICYLQEIFSNEMAKVDDSFFHRLEESFLVEEDKKHERHPIFGNIVDEVAYHEKYPTI
YHLRKKLVDSTDKADLRLIYLALAHMIKFRGHFLIEGDLNPDNSDVDKLFIQLVQTYNQLFEENPIN
ASGVDAKAILSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDEHHQDLTLLK
ALVRQQLPEKYKEIFFDQSKNGYAGYIDGGASQEEFYKFIKPILEKMDGTEELLVKLNREDLLRKQ
RTFDNGSIPHQIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKVKYVTEGMR
KPAFLSGEQKKAIVDLLFKTNRKVTVKQLKEDYFKKIECFDSVEISGVEDRFNASLGTYHDLLKIIK
DKDFLDNEENEDILEDIVLTLTLFEDREMIEERLKTYAHLFDDKVMKQLKRRRYTGWGRLSRKLIN
GIRDKQSGKTILDFLKSDGFANRNFMQLIHDDSLTFKEDIQKAQVSGQGDSLHEHIANLAGSPAIKK
GILQTVKVVDELVKVMGRHKPENIVIEMARENQTTQKGQKNSRERMKRIEEGIKELGSQILKEHPV
ENTQLQNEKLYLYYLQNGRDMYVDQELDINRLSDYDVDAIVPQSFLKDDSIDNKVLTRSDKNRGK
SDNVPSEEVVKKMKNYWRQLLNAKLITQRKFDNLTKAERGGLSELDKAGFIKRQLVETRQITKHV
AQILDSRMNTKYDENDKLIREVKVITLKSKLVSDFRKDFQFYKVREINNYHHAHDAYLNAVVGTA
LIKKYPKLESEFVYGDYKVYDVRKMIAKSEQEIGKATAKYFFYSNIMNFFKTEITLANGEIRKRPLIE
TNGETGEIVWDKGRDFATVRKVLSMPQVNIVKKTEVQTGGFSKESIRPKRNSDKLIARKKDWDPK
KYGGFLWPTVAYSVLVVAKVEKGKSKKLKSVKELLGITIMERSSFEKNPIDFLEAKGYKEVKKDLII
KLPKYSLFELENGRKRMLASAKQLQKGNELALPSKYVNFLYLASHYEKLKGSPEDNEQKQLFVEQ
HKHYLDEIIEQISEFSKRVILADANLDKVLSAYNKHRDKPIREQAENIIHLFTLTRLGAPRAFKYFDT
TIDPKQYRSTKEVLDATLIHQSITGLYETRIDLSQLGGDKRPAATKKAGQAKKKK

MCP (SEQ ID NO:44)
ASNFTQFVLVDNGGTGDVTVAPSNFANGIAEWISSNSRSQAYKVTCSVRQSSAQNRKYTIKVEVPK
GAWRSYLNMELTIPIFATNSDCELIVKAMQGLLKDGNPIPSAIAANSGIY

2×MS2 (SEQ ID NO:45)
GGGAGCACATGAGGATCACCCATGTGCCACGAGCGACATGAGGATCACCCATGTCGCTCGTGT
TCCC

PrimeROOT.v2N-Cre (SEQ ID NO:46)
MPKKKRKVSGGSPKKKRKVSGGSSNLLTVHQNLPALPVDATSDEVRKNLMDMFRDRQAFSEHTW
KMLLSVCRSWAAWCKLNNRKWFPAEPEDVRDYLLYLQARGLAVKTIQQHLGQLNMLHRRSGLPR
PSDSNAVSLVMRRIRKENVDAGERAKQALAFERTDFDQVRSLMENSDRCQDIRNLAFLGIAYNTLL
RIAEIARIRVKDISRTDGGRMLIHIGRTKTLVSTAGVEKALSLGVTKLVERWISVSGVADDPNNYLFC
RVRKNGVAAPSATSQLSTRALEGIFEATHRLIYGAKDDSGQRYLAWSGHSARVGAARDMARAGVS
IPEIMQAGGWTNVNIVMNYIRNLDSETGAMVRLLEDGDSGGSSGGSSGSETPGTSESATPESSGGSS
GGSMPKKKRKVDKKYSIGLDIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHERHPIFGNIV
DEVAYHEKYPTIYHLRKKLVDSTDKADLRLIYLALAHMIKFRGHFLIEGDLNPDNSDVDKLFIQLV
QTYNQLFEENPINASGVDAKAILSARLSKSRRLENLIAQLPGEKKNGLFGNLIALSLGLTPNFKSNF
DLAEDAKLQLSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILEKMDGTEELL
VKLNREDLLRKQRTFDNGSIPHQIHLGELHAILRRQEDFYPFLKDNREKIEKILTFRIPYYVGPLARG
NSRFAWMTRKSEETITPWNFEEVVDKGASAQSFIERMTNFDKNLPNEKVLPKHSLLYEYFTVYNEL
TKVKYVTEGMRKPAFLSGEQKKAIVDLLFKTNRKVTVKQLKEDYFKKIECFDSVEISGVEDRFNAS
LGTYHDLLKIIKDKDFLDNEENEDILEDIVLTLTLFEDREMIEERLKTYAHLFDDKVMKQLKRRRYT
GWGRLSRKLINGIRDKQSGKTILDFLKSDGFANRNFMQLIHDDSLTFKEDIQKAQVSGQGDSLHEHI
ANLAGSPAIKKGILQTVKVVDELVKVMGRHKPENIVIEMARENQTTQKGQKNSRERMKRIEEGIKE
LGSQILKEHPVENTQLQNEKLYLYYLQNGRDMYVDQELDINRLSDYDVDAIVPQSFLKDDSIDNK
VLTRSDKNRGKSDNVPSEEVVKKMKNYWRQLLNAKLITQRKFDNLTKAERGGLSELDKAGFIKR
QLVETRQITKHVAQILDSRMNTKYDENDKLIREVKVITLKSKLVSDFRKDFQFYKVREINNYHHAH
DAYLNAVVGTALIKKYPKLESEFVYGDYKVYDVRKMIAKSEQEIGKATAKYFFYSNIMNFFKTEIT

LANGEIRKRPLIETNGETGEIVWDKGRDFATVRKVLSMPQVNIVKKTEVQTGGFSKESILPKRNSD
KLIARKKDWDPKKYGGFDSPTVAYSVLVVAKVEKGKSKKLKSVKELLGITIMERSSFEKNPIDFLE
AKGYKEVKKDLIIKLPKYSLFELENGRKRMLASAGELQKGNELALPSKYVNFLYLASHYEKLKGS
PEDNEQKQLFVEQHKHYLDEIIEQISEFSKRVILADANLDKVLSAYNKHRDKPIREQAENIIHLFTLT
NLGAPAAFKYFDTTIDRKRYTSTKEVLDATLIHQSITGLYETRIDLSQLGGDEFSGSETPGTSESATPE
SATVVSGQKQDRQGGERRRSQLDRDQCAYCKEKGHWAKDCPKKPRGPRGPRPQTSLLPKKKRKV
ELSGGSSGGSSGSETPGTSESATPESSGGSSGGSRPTLNIEDEYRLHETSKEPDVSLGSTWLSDFPQA
WAETGGMGLAVRQAPLIIPLKATSTPVSIKQYPMSQEARLGIKPHIQRLLDQGILVPCQSPWNTPLLP
VKKPGTNDYRPVQDLREVNKRVEDIHPTVPNPYNLLSGLPPSHQWYTVLDLKDAFFCLRLHPTSQ
PLFAFEWRDPEMGISGQLTWTRLPQGFKNSPTLFNEALHRDLADFRIQHPDLILLQYVDDLLLAATS
ELDCQQGTRALLQTLGNLGYRASAKKAQICQKQVKYLGYLLKEGQRWLTEARKETVMGQPTPKT
PRQLREFLGKAGFCRLFIPGFAEMAAPLYPLTKPGTLFNWGPDQQKAYQEIKQALLTAPALGLPDLT
KPFELFVDEKQGYAKGVLTQKLGPWRRPVAYLSKKLDPVAAGWPPCLRMVAAIAVLTKDAGKLTM
GQPLVILAPHAVEALVKQPPDRWLSNARMTHYQALLLDTDRVQFGPVVALNPATLLPLPEEGLQHN
CLDILAEAHGTRPDLTDQPLSGGSPKKKRKV

PrimeROOT.v2N-FLP (SEQ ID NO:47)
MPKKKRKVSGGSPKKKRKVSGGSSMPQFDILCKTPPKVLVRQFVERFERPSGEKIALCAAELTYLC
WMITHNGTAIKRATFMSYNTIISNSLSFDIVNKSLQFKYKTQKATILEASLKKLIPAWEFTIIPYYGQK
HQSDITDIVSSLQLQFESSEEADKGNSHSKKMLKALLSEGESIWEITEKILNSFEYTSRFTKTKTLYQ
FLFLATFINCGRFSDIKNVDPKSFKLVQNKYLGVIIQCLVTETKTSVSRHIYFFSARGRIDPLVYLDEF
LRNSEPVLKRVNRTGNSSSNKQEYQLLKDNLVRSYNKALKKNAPYSIFAIKNGPKSHIGRHLMTSF
LSMKGLTELTNVVGNWSDKRASAVARTTYTHQITAIPDHYFALVSRYYAYDPISKEMIALKDETNPI
EEWQHIEQLKGSAEGSIRYPAWNGIISQEVLDYLSSYINRRISGGSSGGSSGSETPGTSESATPESSGG
SSGGSMPKKKRKVDKKYSIGLDIGTNSVGWAVITDEYKVPSKKFKVLGNTDRHSIKKNLIGALLFD
SGETAEATRLKRTARRRYTRRKNRICYLQEIFSNEMAKVDDSFFHRLEESFLVEEDKKHERHPIFGNI
VDEVAYHEKYPTIYHLRKKLVDSTDKADLRLIYLALAHMIKFRGHFLIEGDLNPDNSDVDKLFIQL
VQTYNQLFEENPINASGVDAKAILSARLSKSRRLENLIAQLPGEKKNGLFGNLIALSLGLTPNFKSN
FDLAEDAKLQLSKDTYDDDLDNLLAQIGDQYADLFLAAKNLSDAILLSDILRVNTEITKAPLSASMI
KRYDEHHQDLTLLKALVRQQLPEKYKEIFFDQSKNGYAGYIDGGASQEEFYKFIKPILEKMDGTEE
LLVKLNREDLLRKQRTFDNGSIPHQIHLGELHAILRRQEDFYPFLKDNREKIEKILTFRIPYYVGPLA
RGNSRFAWMTRKSEETITPWNFEEVVDKGASAQSFIERMTNFDKNLPNEKVLPKHSLLYEYFTVY
NELTKVKYVTEGMRKPAFLSGEQKKAIVDLLFKTNRKVTVKQLKEDYFKKIECFDSVEISGVEDRF
NASLGTYHDLLKIIKDKDFLDNEENEDILEDIVLTLTLFEDREMIEERLKTYAHLFDDKVMKQLKRR
RYTGWGRLSRKLINGIRDKQSGKTILDFLKSDGFANRNFMQLIHDDSLTFKEDIQKAQVSGQGDSL
HEHIANLAGSPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDAIVPQSFLKDDSI
DNKVLTRSDKNRGKSDNVPSEEVVKKMKNYWRQLLNAKLITQRKFDNLTKAERGGLSELDKAGF
IKRQLVETRQITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSEQEIGKATAKYFFYSNIMNFFKTE
ITLANGEIRKRPLIETNGETGEIVWDKGRDFATVRKVLSMPQVNIVKKTEVQTGGFSKESILPKRNS
DKLIARKKDWDPKKYGGFDSPTVAYSVLVVAKVEKGKSKKLKSVKELLGITIMERSSFEKNPIDFL
EAKGYKEVKKDLIIKLPKYSLFELENGRKRMLASAGELQKGNELALPSKYVNFLYLASHYEKLKG
SPEDNEQKQLFVEQHKHYLDEIIEQISEFSKRVILADANLDKVLSAYNKHRDKPIREQAENIIHLFTL
TNLGAPAAFKYFDTTIDRKRYTSTKEVLDATLIHQSITGLYETRIDLSQLGGDEFSGSETPGTSESATP
ESATVVSGQKQDRQGGERRRSQLDRDQCAYCKEKGHWAKDCPKKPRGPRGPRPQTSLLPKKKRK
VELSGGSSGGSSGSETPGTSESATPESSGGSSGGSRPTLNIEDEYRLHETSKEPDVSLGSTWLSDFPQ
AWAETGGMGLAVRQAPLIIPLKATSTPVSIKQYPMSQEARLGIKPHIQRLLDQGILVPCQSPWNTPLL
PVKKPGTNDYRPVQDLREVNKRVEDIHPTVPNPYNLLSGLPPSHQWYTVLDLKDAFFCLRLHPTS
QPLFAFEWRDPEMGISGQLTWTRLPQGFKNSPTLFNEALHRDLADFRIQHPDLILLQYVDDLLLAA
TSELDCQQGTRALLQTLGNLGYRASAKKAQICQKQVKYLGYLLKEGQRWLTEARKETVMGQPTP
KTPRQLREFLGKAGFCRLFIPGFAEMAAPLYPLTKPGTLFNWGPDQQKAYQEIKQALLTAPALGLPD
LTKPFELFVDEKQGYAKGVLTQKLGPWRRPVAYLSKKLDPVAAGWPPCLRMVAAIAVLTKDAGKL
TMGQPLVILAPHAVEALVKQPPDRWLSNARMTHYQALLLDTDRVQFGPVVALNPATLLPLPEEGLQ
HNCLDILAEAHGTRPDLTDQPLSGGSPKKKRKV

PrimeROOT.v2C-Cre (SEQ ID NO:48)
MPKKKRKVDKKYSIGLDIGTNSVGWAVITDEYKVPSKKFKVLGNTDRHSIKKNLIGALLFDSGETA
EATRLKRTARRRYTRRKNRICYLQEIFSNEMAKVDDSFFHRLEESFLVEEDKKHERHPIFGNIVDEV
AYHEKYPTIYHLRKKLVDSTDKADLRLIYLALAHMIKFRGHFLIEGDLNPDNSDVDKLFIQLVQTY

NQLFEENPINASGVDAKAILSARLSKSRRLENLIAQLPGEKKNGLFGNLIALSLGLTPNFKSNFDLAE
DAKLQLSKDTYDDDLDNLLAQIGDQYADLFLAAKNLSDAILLSDILRVNTEITKAPLSASMIKRYD
EHHQDLTLLKALVRQQLPEKYKEIFFDQSKNGYAGYIDGGASQEEFYKFIKPILEKMDGTEELLVKL
NREDLLRKQRTFDNGSIPHQIHLGELHAILRRQEDFYPFLKDNREKIEKILTFRIPYYVGPLARGNSR
FAWMTRKSEETITPWNFEEVVDKGASAQSFIERMTNFDKNLPNEKVLPKHSLLYEYFTVYNELTKV
KYVTEGMRKPAFLSGEQKKAIVDLLFKTNRKVTVKQLKEDYFKKIECFDSVEISGVEDRFNASLGT
YHDLLKIIKDKDFLDNEENEDILEDIVLTLTLFEDREMIEERLKTYAHLFDDKVMKQLKRRRYTGW
GRLSRKLINGIRDKQSGKTILDFLKSDGFANRNFMQLIHDDSLTFKEDIQKAQVSGQGDSLHEHIAN
LAGSPAIKKGILQTVKVVDELVKVMGRHKPENIVIEMARENQTTQKGQKNSRERMKRIEEGIKELG
SQILKEHPVENTQLQNEKLYLYYLQNGRDMYVDQELDINRLSDYDVDAIVPQSFLKDDSIDNKVLT
RSDKNRGKSDNVPSEEVVKKMKNYWRQLLNAKLITQRKFDNLTKAERGGLSELDKAGFIKRQLV
ETRQITKHVAQILDSRMNTKYDENDKLIREVKVITLKSKLVSDFRKDFQFYKVREINNYHHAHDAY
LNAVVGTALIKKYPKLESEFVYGDYKVYDVRKMIAKSEQEIGKATAKYFFYSNIMNFFKTEITLAN
GEIRKRPLIETNGETGEIVWDKGRDFATVRKVLSMPQVNIVKKTEVQTGGFSKESILPKRNSDKLIA
RKKDWDPKKYGGFDSPTVAYSVLVVAKVEKGKSKKLKSVKELLGITIMERSSFEKNPIDFLEAKGY
KEVKKDLIIKLPKYSLFELENGRKRMLASAGELQKGNELALPSKYVNFLYLASHYEKLKGSPEDNE
QKQLFVEQHKHYLDEIIEQISEFSKRVILADANLDKVLSAYNKHRDKPIREQAENIIHLFTLTNLGAP
AAFKYFDTTIDRKRYTSTKEVLDATLIHQSITGLYETRIDLSQLGGDEFSGSETPGTSESATPESATVV
SGQKQDRQGGERRRSQLDRDQCAYCKEKGHWAKDCPKKPRGPRGPRPQTSLLPKKKRKVELSGG
SSGGSSGSETPGTSESATPESSGGSSGGSRPTLNIEDEYRLHETSKEPDVSLGSTWLSDFPQAWAETG
GMGLAVRQAPLIIPLKATSTPVSIKQYPMSQEARLGIKPHIQRLLDQGILVPCQSPWNTPLLPVKKPG
TNDYRPVQDLREVNKRVEDIHPTVPNPYNLLSGLPPSHQWYTVLDLKDAFFCLRLHPTSQPLFAFE
WRDPEMGISGQLTWTRLPQGFKNSPTLFNEALHRDLADFRIQHPDLILLQYVDDLLLAATSELDCQ
QGTRALLQTLGNLGYRASAKKAQICQKQVKYLGYLLKEGQRWLTEARKETVMGQPTPKTPRQLR
EFLGKAGFCRLFIPGFAEMAAPLYPLTKPGTLFNWGPDQQKAYQEIKQALLTAPALGLPDLTKPFEL
FVDEKQGYAKGVLTQKLGPWRRPVAYLSKKLDPVAAGWPPCLRMVAAIAVLTKDAGKLTMGQPL
VILAPHAVEALVKQPPDRWLSNARMTHYQALLLDTDRVQFGPVVALNPATLLPLPEEGLQHNCLDI
LAEAHGTRPDLTDQPLSGGSPKKKRKVSGGSSGGSSGSETPGTSESATPESSGGSSGGSPKKKRKVS
GGSSNLLTVHQNLPALPVDATSDEVRKNLMDMFRDRQAFSEHTWKMLLSVCRSWAAWCKLNNR
KWFPAEPEDVRDYLLYLQARGLAVKTIQQHLGQLNMLHRRSGLPRPSDSNAVSLVMRRIRKENVD
AGERAKQALAFERTDFDQVRSLMENSDRCQDIRNLAFLGIAYNTLLRIAEIARIRVKDISRTDGGRM
LIHIGRTKTLVSTAGVEKALSLGVTKLVERWISVSGVADDPNNYLFCRVRKNGVAAPSATSQLSTRA
LEGIFEATHRLIYGAKDDSGQRYLAWSGHSARVGAARDMARAGVSIPEIMQAGGWTNVNIVMNYI
RNLDSETGAMVRLLEDGDKRPAATKKAGQAKKKK

PrimeROOT.v2C-FLP (SEQ ID NO:49)
MPKKKRKVDKKYSIGLDIGTNSVGWAVITDEYKVPSKKFKVLGNTDRHSIKKNLIGALLFDSGETA
EATRLKRTARRRYTRRKNRICYLQEIFSNEMAKVDDSFFHRLEESFLVEEDKKHERHPIFGNIVDEV
AYHEKYPTIYHLRKKLVDSTDKADLRLIYLALAHMIKFRGHFLIEGDLNPDNSDVDKLFIQLVQTY
NQLFEENPINASGVDAKAILSARLSKSRRLENLIAQLPGEKKNGLFGNLIALSLGLTPNFKSNFDLAE
DAKLQLSKDTYDDDLDNLLAQIGDQYADLFLAAKNLSDAILLSDILRVNTEITKAPLSASMIKRYD
EHHQDLTLLKALVRQQLPEKYKEIFFDQSKNGYAGYIDGGASQEEFYKFIKPILEKMDGTEELLVKL
NREDLLRKQRTFDNGSIPHQIHLGELHAILRRQEDFYPFLKDNREKIEKILTFRIPYYVGPLARGNSR
FAWMTRKSEETITPWNFEEVVDKGASAQSFIERMTNFDKNLPNEKVLPKHSLLYEYFTVYNELTKV
KYVTEGMRKPAFLSGEQKKAIVDLLFKTNRKVTVKQLKEDYFKKIECFDSVEISGVEDRFNASLGT
YHDLLKIIKDKDFLDNEENEDILEDIVLTLTLFEDREMIEERLKTYAHLFDDKVMKQLKRRRYTGW
GRLSRKLINGIRDKQSGKTILDFLKSDGFANRNFMQLIHDDSLTFKEDIQKAQVSGQGDSLHEHIAN
LAGSPAIKKGILQTVKVVDELVKVMGRHKPENIVIEMARENQTTQKGQKNSRERMKRIEEGIKELG
SQILKEHPVENTQLQNEKLYLYYLQNGRDMYVDQELDINRLSDYDVDAIVPQSFLKDDSIDNKVLT
RSDKNRGKSDNVPSEEVVKKMKNYWRQLLNAKLITQRKFDNLTKAERGGLSELDKAGFIKRQLV
ETRQITKHVAQILDSRMNTKYDENDKLIREVKVITLKSKLVSDFRKDFQFYKVREINNYHHAHDAY
LNAVVGTALIKKYPKLESEFVYGDYKVYDVRKMIAKSEQEIGKATAKYFFYSNIMNFFKTEITLAN
GEIRKRPLIETNGETGEIVWDKGRDFATVRKVLSMPQVNIVKKTEVQTGGFSKESILPKRNSDKLIA
RKKDWDPKKYGGFDSPTVAYSVLVVAKVEKGKSKKLKSVKELLGITIMERSSFEKNPIDFLEAKGY
KEVKKDLIIKLPKYSLFELENGRKRMLASAGELQKGNELALPSKYVNFLYLASHYEKLKGSPEDNE
QKQLFVEQHKHYLDEIIEQISEFSKRVILADANLDKVLSAYNKHRDKPIREQAENIIHLFTLTNLGAP
AAFKYFDTTIDRKRYTSTKEVLDATLIHQSITGLYETRIDLSQLGGDEFSGSETPGTSESATPESATVV
SGQKQDRQGGERRRSQLDRDQCAYCKEKGHWAKDCPKKPRGPRGPRPQTSLLPKKKRKVELSGG
SSGGSSGSETPGTSESATPESSGGSSGGSRPTLNIEDEYRLHETSKEPDVSLGSTWLSDFPQAWAETG
GMGLAVRQAPLIIPLKATSTPVSIKQYPMSQEARLGIKPHIQRLLDQGILVPCQSPWNTPLLPVKKPG

TNDYRPVQDLREVNKRVEDIHPTVPNPYNLLSGLPPSHQWYTVLDLKDAFFCLRLHPTSQPLFAFE
WRDPEMGISGQLTWTRLPQGFKNSPTLFNEALHRDLADFRIQHPDLILLQYVDDLLLAATSELDCQ
QGTRALLQTLGNLGYRASAKKAQICQKQVKYLGYLLKEGQRWLTEARKETVMGQPTPKTPRQLR
EFLGKAGFCRLFIPGFAEMAAPLYPLTKPGTLFNWGPDQQKAYQEIKQALLTAPALGLPDLTKPFEL
FVDEKQGYAKGVLTQKLGPWRRPVAYLSKKLDPVAAGWPPCLRMVAAIAVLTKDAGKLTMGQPL
VILAPHAVEALVKQPPDRWLSNARMTHYQALLLDTDRVQFGPVVALNPATLLPLPEEGLQHNCLDI
LAEAHGTRPDLTDQPLSGGSPKKKRKVSGGSSGGSSGSETPGTSESATPESSGGSSGGSPKKKRKVS
GGSSMPQFDILCKTPPKVLVRQFVERFERPSGEKIALCAAELTYLCWMITHNGTAIKRATFMSYNTII
SNSLSFDIVNKSLQFKYKTQKATILEASLKKLIPAWEFTIIPYYGQKHQSDITDIVSSLQLQFESSEEA
DKGNSHSKKMLKALLSEGESIWEITEKILNSFEYTSRFTKTKTLYQFLFLATFINCGRFSDIKNVDPK
SFKLVQNKYLGVIIQCLVTETKTSVSRHIYFFSARGRIDPLVYLDEFLRNSEPVLKRVNRTGNSSSNK
QEYQLLKDNLVRSYNKALKKNAPYSIFAIKNGPKSHIGRHLMTSFLSMKGLTELTNVVGNWSDKR
ASAVARTTYTHQITAIPDHYFALVSRYYAYDPISKEMIALKDETNPIEEWQHIEQLKGSAEGSIRYPA
WNGIISQEVLDYLSSYINRRIKRPAATKKAGQAKKKK

B2 recombinase amino acid sequence (SEQ ID NO:50)
MSEFSELVRILPLDQVAEIKRILSRGDPIPLQRLASLLTMVILTVNMSKKRKSSPIKLSTFTKYRRNVA
KSLYYDMSSKTVFFEYHLKNTQDLQEGLEQAIAPYNFVVKVHKKPIDWQKQLSSVHERKAGHRSI
LSNNVGAEISKLAETKDSTWSFIERTMDLIEARTRQPTTRVAYRFLLQLTFMNCCRANDLKNADPST
FQIIADPHLGRILRAFVPETKTSIERFIYFFPCKGRCDPLLALDSYLLWVGPVPKTQTTDEETQYDYQ
LLQDTLLISYDRFIAKESKENIFKIPNGPKAHLGRHLMASYLGNNSLKSEATLYGNWSVERQEGVS
KMADSRYMHTVKKSPPSYLFAFLSGYYKKSNQGEYVLAETLYNPLDYDKTLPITTNEKLICRRYG
KNAKVIPKDALLYLYTYAQQKRKQLADPNEQNRLFSSESPAHPFLTPQSTGSSTPLTWTAPKTLSTG
LMTPGEE

KD recombinase amino acid sequence (SEQ ID NO:51)
MSTFAEAAHLTPHQCANEINEILESDTFNINAKEIRNKLASLFSILTMQSLSIRREMKINTYRSYKSAI
GKSLSFDKDDKIIKFTVRLRKTESLQKDIESALPSYKVVVSPFKNQEVSLFDRYEETHKYDASMVG
LQFTNILSKEKDIWKIVSRIACFFDQSCVTTTKRAEYRLLLLGAVGNCCRYSDLKNLDPRTFEIYNN
SFLGPIVRATVTETKSRTERYVNFYPVNGDCDLLISLYDYLRVCSPIEKTVSSNRPTNQTHQFLPESL
ARTFSRFLTQHVDEPVFKIWNGPKSHFGRHLMATFLSRSEKGKYVSSLGNWAGDREIQSAVARSHY
SHGSVTVDDRVFAFISGFYKEAPLGSEIYVLKDPSNKPLSREELLEEEGNSLGSPPLSPPSSPRLVAQS
FSAHPSLQLFEQWHGIISDEVLQFIAEYRRKHELRSQRTVVA

pSR1 recombinase amino acid sequence (SEQ ID NO:52)
MQLTKDTEISTINRQMSDFSELSQILPLHQISKIKDILENENPLPKEKLASHLTMIILMANLASQKRKD
VPVKRSTFLKYQRSISKTLQYDSSTKTVSFEYHLKDPSKLIKGLEDVVSPYRFVVGVHEKPDDVMS
HLSAVHMRKEAGRKRDLGNKINDEITKIAETQETIWGFVGKTMDLIEARTTRPTTKAAYNLLLQAT
FMNCCRADDLKNTDIKTFEVIPDKHLGRMLRAFVPETKTGTRFVYFFPCKGRCDPLLALDSYLQW
TDPIPKTRTTDEDARYDYQLLRNSLLGSYDGFISKQSDESIFKIPNGPKAHLGRHVTASYLSNNEMD
KEATLYGNWSAAREEGVSRVAKARYMHTIEKSPPSYLFAFLSGFYNITAERACELVDPNSNPCEQD
KNIPMISDIETLMARYGKNAEIIPMDVLVFLSSYARFKNNEGKEYKLQARSSRGVPDFPDNGRTALY
NALTAAHVKRRKISIVVGRSIDTS

B2 recombinase recognition site (SEQ ID NO:53)
GAGTTTCATTAAGGAATAACTAATTCCCTAATGAAACTC

KD recombinase recognition site (SEQ ID NO:54)
AAACGATATCAGACATTTGTCTGATAATGCTTCATTATCAGACAAATGTCTGATATCGTTT

pSR1 recombinase recognition site (SEQ ID NO:55)
TTGATGAAAGAATAACGTATTCTTTCATCAA

Dre recombinase amino acid sequence （SEQ ID NO:56）

MSELIISGSSGGFLRNIGKEYQEAAENFMRFMNDQGAYAPNTLRDLRLVFHSWARWCHARQLAWF
PISPEMAREYFLQLHDADLASTTIDKHYAMLNMLLSHCGLPPLSDDKSVSLAMRRIRREAATEKGE
RTGQAIPLRWDDLKLLDVLLSRSERLVDLRNRAFLFVAYNTLMRMSEISRIRVGDLDQTGDTVTLHI
SHTKTITTAAGLDKVLSRRTTAVLNDWLDVSGLREHPDAVLFPPIHRSNKARITTTPLTAPAMEKIFS
DAWVLLNKRDATPNKGRYRTWTGHSARVGAAIDMAEKQVSMVEIMQEGTWKKPETLMRYLRRG

GVSVGANSRLMDS

Dre recombinase recognition site rox（SEQ ID NO:57）
TAACTTTAAATAATGCCAATTATTTAAAGTTA

Dre recombinase recognition site rox（SEQ ID NO:58）
TAACTTTAAATAATGTCCATTATTTAAAGTTA

Fig. 14A PrimeROOT.v2-Cre insertion S20-T2 (SEQ ID NO:59)
GATCCTGTGCAATTTGAAAGGAACCCTGACGAGATTCCGTGGGCTGAATAACTTCGTATAGCAT
ACATTATACGAAGTTATTCGAGGTCATTCATAT

Fig. 14A PrimeROOT.v2-Cre insertion S20-T4 (SEQ ID NO:60)
ATACCTTCAAGTGAGCAGCAGCCTTCTCCTTGTCAGTGAAGACACTACCGTTCGTATAATGTATG
CTATACGAACGGTAGGTCTACCTAC

Fig. 14A NHEJ insertion S20-T2 (SEQ ID NO:61)
GATCCTGTGCAATTTGAAAGGAACCCTGACGAGATTCCGTGGGCTGAGGGTGGGCTTGGCTTT
GTTTTCGGTCTCCGCCCCCCCGGGCGTTTTTATG

Fig. 14A NHEJ insertion S20-T4 (SEQ ID NO:62)
CAGTGAAGACACCGGTGGACTCCACGACATACTCAGCACCAGCCGGGTGGGCGGGACCTCTTC
TACCTACAAAAAAGCTCCGCACGA

Fig. 14A NHEJ insertion S20-T2 -80bp (SEQ ID NO:63)
CGTGGAACTGATGTTT//GA…//…//AAGGTGGTATA

Fig. 14A NHEJ insertion S20-T2 +35bp (SEQ ID NO:64)
CGTGGAACTGATGTTT//ATGGCTGGGCTTGGCCTTGAATTCGAGCTCGGTACCCTCGA//TCAGTT
AAAAGGTGGTATA

Fig. 14A NHEJ insertion S20-T2 +34/-1bp (SEQ ID NO:65)
CGTGGAACTGATGTTT//GCTGGGCTTGGCCTTGAATTCGAGCTCGGTACCC-
TCGAGG//TCAGTTAAAAGGTGGTATA

Fig. 14A NHEJ insertion S20-T2 +44/-79bp (SEQ ID NO:66)
CGTGGAACTGATGTTTCAGTA//CGGA…//…AAAAGAGTTG//AAGGTGGTATA

Fig. 14A NHEJ insertion S20-T2 +62/-4bp (SEQ ID NO:67)
CGTGGAACTGATGTTT//GAGGGAGAGGCGGTG//GCTCGCTGCGCTC …
GGTCGTTCA//AGTTAAAAGGTGGTATA

Fig. 14A NHEJ insertion S20-T2 +289/-1bp (SEQ ID NO:68)
CGTGGAACTGATGTTT//TGCGTTTCTGGGTGAG//TCGAGCTCGGTACCC-
TCGAGGTC//CAGTTAAAAGGTGGTATA

Fig. 14A NHEJ insertion S20-T2 +271/-7bp (SEQ ID NO:69)
CGTGGAACTGATGTTT//TGGTCGTTCGCTCCAAGC//CTCGAGGTCAT …
TCGAGGTC//TAAAAGGTGGTATA

Fig. 14A NHEJ insertion S20-T2 +151bp (SEQ ID NO:70)
CGTGGAACTGATGTTT//GAGTTTTCGTTCCACTGACT//TAATTCGAGCTCGGTACCCCG//AGTTA
AAAGGTGGTATA

Fig. 14A NHEJ insertion S20-T2 +72bp (SEQ ID NO:71)
CGTGGAACTGATGTTT//TGAGGTAAGATTACCTGGTC//GAATTCGAGCTCGGTACCCTC//AGTTA
AAAGGTGGTATA

Fig. 14A NHEJ insertion S20-T2 +183/-92bp (SEQ ID NO:72)

CGTGG//AA-A//ATCC//AAAT…//…//AAGGTGGTATA

Fig. 14A NHEJ insertion S20-T4 +15bp (1) (SEQ ID NO:73)
CTTTTCATGATTTGTGACAAATGCAGC//GAAGAGGTACCGGCCAGCCGGCTGGTGCTGAGTATG
TCGTGGAGTCCACCGG

Fig. 14A NHEJ insertion S20-T4 +12bp (SEQ ID NO:74)
CTTTTCATGATTTGTGACAAATGCAGCCT/AGAAGAGGTACCGGCCAGCCTGGTGCTGAGTATGT
CGTGGAGTCCACCGG

Fig. 14A NHEJ insertion S20-T4 +109/-2bp (SEQ ID NO:75)
CCTTTCATGATTTGTGACAAATGC//GAAGAGGTAT//CTGCGTTA--
GCCGGTGCTGAGTATGTCGTGGAGTCCACCGG

Fig. 14A NHEJ insertion S20-T4 +15bp (2) (SEQ ID NO:76)
CTTTTCACGATTTGTGACAAATGCAGC//GAAGAGGTACCGGCCAGCCGGCTGGTGCTGAGTGT
GTCGTGGAGTCCACCGG

Fig. 14A NHEJ insertion S20-T4 +172/-5bp (SEQ ID NO:77)
CTTTTCATGATTTGTGACAAATG//GAAGAGGTAC//AGACCCCGT-----
TGGTGCTGAGTATGTCGTGGAGTCCACCGG

Fig. 14A NHEJ insertion S20-T4 +15bp (3) (SEQ ID NO:78)
CTTTTCACGATTTGTGACAAATGCAGC//GAAGAGGTACCGGCCAGCCGGCTGGTGCTGAGTGT
GTCGTGGAGTCCACCGG

Fig. 14A NHEJ insertion S20-T4 +47/-2bp (SEQ ID NO:79)
CTTTTCATGATTTGTGACAAATGC//GAAGAGGTACC//AGCTCGG--
GGCTGGTGCTGAGTATGTCGTGGAGTCCACCGG

Fig. 14A NHEJ insertion S20-T4 +143/-2bp (SEQ ID NO:80)
CTTTTCATGATTTGTGGCAAATGC//GAAGAGGTACC//CCAGCCT--
GGCTGGTGCTGAGTATGTCGTGGAGTCCACCGG

Fig. 14A NHEJ insertion S20-T4 +15bp (4) (SEQ ID NO:81)
CTTTTCATGATTTGTGACAAATGCAGC//GAAGAGGTACCGGCCAGTCGGCTGGTGCTGAGTATG
TCGTGGAGTCCACCGG

Fig. 14A NHEJ insertion S20-T4 +15bp (5) (SEQ ID NO:82)
CTTTTCATGATTTGTGACAAATGCATC/AAAGAGGTACCGGCCAGCCGGCTGGCGCTGAGTATGT
CGAGGAGTCCACCGG

Fig. 14C PrimeROOT.v2C-Cre-Act1P or PrimeROOT.v2C-Act1P-pigmT insertion (SEQ ID NO:83)
GAAGCATCTGTCTGTCCACTCCCCACTCGTATAACTTCGTATAGCATACATTATACGAA

Fig. 14C NHEJ strategy-ActP insertion ins 36bp (SEQ ID NO:84)
CTCCCACTCGTTGCACGGGCTTGGC//TTCGAGCTCGGTACCCTCGAGGTCATTCATA

Fig. 14C NHEJ strategy-ActP insertion ins 12/del 1bp (SEQ ID NO:85)
ATGTCTGTCCACTCCCCACTCGAGCTCGGTACCC-TCGAGGTCATTCATATGCTTGAGA

Fig. 14C NHEJ strategy-ActP insertion ins 131bp (SEQ ID NO:86)
CTCCCCACTCGTTTTTCCGAAGGTAAC//CGAGCTCGGTACCCTTCGAGGTCATTCATA

Fig. 14C NHEJ strategy-ActP-pigmR insertion ins 38/del 11bp (SEQ ID NO:87)
CTCCCCACTGCTTGGC//CCTCCCTGC-----------CATTCATATGCTTGAGA

Fig. 18B GFP-N terminal-hLMNB1-F (SEQ ID NO:88)
ACGGCATGGACGAGCTGTACAAGTAATTTTTTTTACCGTTCGTATAGCATACATTATACGAACGG
TAAGCCCCACGCGCCTGTCGCGGCTCCAGGAGAAGGAGGAGCTGCGCGAGCTCAATGAC

Fig. 18B GFP-C terminal -hLMNB1-R (SEQ ID NO:89)
CCCGGTGAACAGCTCCTCGCCCTTGCTCACCATATAACTTCGTATAATGTATGCTATACGAAGTTA
TCGGGCGGCGGAGACAGCGGGGCGGCGAGGCCGCGAGCGGGACCGTGATAAGGAG

## Claims

1. A genome editing system for inserting an exogenous nucleotide sequence in a plant genome, comprising:
i) a) a CRISPR nuclease and/or an expression construct comprising a nucleotide sequence encoding said CRISPR nuclease, and a reverse transcriptase and/or an expression construct comprising a nucleotide sequence encoding said reverse transcriptase ,or
b) a prime editing fusion protein and/or an expression construct comprising a nucleotide sequence encoding the prime editing fusion protein, wherein the prime editing fusion protein comprises CRISPR nuclease and reverse transcriptase;
ii) a first pegRNA and/or an expression construct comprising a nucleotide sequence encoding said first pegRNA, and
iii) a second pegRNA and/or an expression construct containing a nucleotide sequence encoding the second pegRNA,
wherein the first pegRNA comprises a first prime sequence, a first scaffold (scaffold) sequence, a first reverse transcription template (RT) sequence and a first primer binding site (PBS) sequence from 5' to 3' direction,
wherein the second pegRNA comprises a second prime sequence, a first scaffold (scaffold) sequence, a second reverse transcription template (RT) sequence and a second primer binding site (PBS) sequence from 5' to 3' direction,
wherein the first pegRNA targets a first target sequence on the sense strand of plant genomic DNA, and the second pegRNA targets a second target sequence on the antisense strand of plant genomic DNA,
wherein the first RT sequence and the second RT sequence are used to insert the first foreign nucleotide sequence.

2. The genome editing system of claim 1, wherein said pegRNA is capable of forming a complex with said CRISPR nuclease or fusion protein and targeting said CRISPR nuclease or fusion protein to a target sequence in the genome, resulting in a nick within the target sequence on said target strand.

3. The genome editing system according to claim 1 or 2, wherein the interval between the PAMs of the first target sequence and the second target sequence is about 20bp-about 80bp, such as about 20bp-about 60bp.

4. The genome editing system of any one of claims 1-3, wherein the CRISPR nuclease is a Cas9 nuclease or a variant thereof.

5. The genome editing system of any one of claims 1-4, wherein the CRISPR nuclease is a CRISPR nickase, such as a Cas9 nickase or a variant thereof, such as the Cas9 nickase or a variant thereof comprising a sequence selected from SEQ ID NOs: 2 and 42-43.

6. The genome editing system according to any one of claims 1-5, wherein said CRISPR nuclease such as Cas9 nickase and said reverse transcriptase are connected by a linker.

7. The genome editing system according to any one of claims 1-6, wherein the reverse transcriptase is M-MLV reverse transcriptase or a functional variant thereof.

8. The genome editing system according to any one of claims 1-7, wherein the RNase H domain of the reverse transcriptase such as M-MLV reverse transcriptase or a functional variant thereof is deleted.

9. The genome editing system according to any one of claims 1-8, wherein reverse transcriptase such as M-MLV reverse transcriptase or its functional variant is fused directly or by linker.

10. The genome editing system of claim 9, wherein the nucleocapsid protein (NC) comprises the amino acid sequence shown in SEQ ID NO:6.

11. The genome editing system according to any one of claims 1-10, wherein the reverse transcriptase is fused with an RNA aptamer binding protein sequence such as an MCP protein sequence through a linker or directly, and the pegRNA comprises one or more RNA aptamer sequences such as MS2 sequences.

12. The genome editing system according to any one of claims 1-11, wherein said CRISPR nuclease such as a CRISPR nickase in i)-b) is fused to said reverse transcriptase via a self-cleavable peptide.

13. The genome editing system according to any one of claims 1-12, wherein said CRISPR nuclease such as a CRISPR nickase in i)-b) is fused to the N-terminus of said reverse transcriptase.

14. The genome editing system according to any one of claims 1-13, wherein the fusion protein in i)-b) comprises the amino acid sequence shown in SEQ ID NO:19.

15. The genome editing system according to any one of claims 1-14, whereinthe prime sequence in the first pegRNA may have sufficient sequence identity (preferably 100% identity) to the first target sequence such that the first pegRNA's complex with a CRISPR nuclease such as a nickase results in a nick in the first target sequence; the prime sequence in the second pegRNA may have sufficient sequence identity (preferably 100% identity) to the second target sequence on the opposite strand such that the second pegRNA's complex with a CRISPR nuclease such as a nickase results in a nick in the second target sequence.

16. The genome editing system according to any one of claims 1-15, wherein the scaffold sequence of the gRNA is shown in SEQ ID NO:17.

17. The genome editing system according to any one of claims 1-16, wherein the primer binding sequence is configured to be complementary to at least a part of the target sequence (preferably perfectly paired with at least a part of the target sequence), preferably, the primer binding sequence is complementary to at least a part of the 3' free single strand resulted from the nick in the DNA chain where the target sequence is located (preferably perfectly paired with at least a part of the 3' free single strand), especially complementary to the nucleotide sequence at the 3' end of the 3' free single strand (preferably perfect pairing).

18. The genome editing system according to any one of claims 1-17, wherein the RT sequence is configured to generate an exogenous nucleotide sequence or part thereof to be inserted into the genome after reverse transcription using it as a template, or to generate a complement of the exogenous nucleotide sequence or a portion thereof to be inserted into the genome of an organism such as a plant.

19. The genome editing system according to any one of claims 1-18, wherein the first RT sequence and the second RT sequence are used to insert a first exogenous nucleotide sequence, for example, to insert a first exogenous nucleotide sequence between the first target sequence and the second target sequence, preferably, the first RT sequence of the first pegRNA is configured to generate a first fragment of the first exogenous nucleotide sequence to be inserted into the genome after reverse transcription using it as a template; the second RT sequence of the second pegRNA is configured to generate the complementary sequence of a second fragment of the first exogenous nucleotide sequence to be inserted into the genome after reverse transcription using it as a template

20. The genome editing system of claim 19, wherein the first segment and the second segment of the first exogenous nucleotide sequence to be inserted at least partially overlap.

21. The genome editing system of claim 20, wherein the first fragment and the second fragment overlap by at least about 10 bp to about 50 bp.

22. The genome editing system according to any one of claims 1-21, the pegRNA also comprises a tevopre sequence at the 3' end of PBS.

23. The genome editing system according to any one of claims 1-22, the pegRNA also comprises a polyA sequence at the 3' end.

24. The genome editing system according to any one of claims 1-23, the length of the first foreign nucleotide sequence to be inserted is about 1 bp to about 700 bp.

25. The genome editing system according to any one of claims 1-24, the 5' end of the pegRNA is connected to a first ribozyme or tRNA, and the first ribozyme or tRNA is designed to be at the 5' end of the pegRNA and/or the 3' end of the pegRNA is linked to a second ribozyme or tRNA designed to cleave the fusion at the 3' end of the pegRNA.

26. The genome editing system according to any one of claims 1-25, wherein the pegRNA is transcribed by a type II promoter, for example, the type II promoter is a GS promoter.

27. The genome editing system according to any one of claims 1-26, wherein the first exogenous nucleotide sequence comprises one or more recognition sites (RS) for recombinases.

28. The genome editing system of claim 27, further comprising:
iv) a recombinase and/or an expression construct comprising a nucleotide sequence encoding said recombinase, and
v) a donor construct comprising one or more recognition sites (RS) for said recombinase and a second exogenous polynucleotide sequence to be inserted into the plant genome.

29. The genome editing system of claim 27 or 28, wherein the recombinase is a recombinase of the tyrosine recombinase family or a recombinase of the serine recombinase family, preferably a recombinase of the tyrosine recombinase family,
for example, the recombinase of the tyrosine recombinase family is selected from B2, KD, pSR1, Dre, Cre, FLP; or the recombinase of the serine recombinase family is selected from phiC31 and Bxb1.

30. The genome editing system of any one of claims 27-29, wherein the recombinase is Cre recombinase.

31. The genome editing system of claim 30, wherein the one or more recombinase recognition sites (RS) are selected from loxP, Lox2272, Lox71, Lox66, or variants thereof, and any combination thereof.

32. The genome editing system of any one of claims 27-29, wherein the recombinase is FLP recombinase.

33. The genome editing system of claim 32, said one or more recombinase recognition sites (RS) selected from FRT1, FRT3, FRT5, FRT6 or their variants as comprising one of SEQ ID NO:32-37 FRT1 variants of the above sequences, and any combination thereof.

34. The genome editing system according to any one of claims 28-33, wherein said recombinase is contained in said guided editing fusion protein of i)-b),
for example, the recombinase is located at the N-terminal or C-terminal of the fusion protein, which is connected directly or through a linker to other parts of the fusion protein,
preferably, the guide editing fusion protein comprises the amino acid sequence shown in any one of SEQ ID NO: 46-49 or an amino acid sequence having 85%, 90%, or 95% identity therewith.

35. The genome editing system of any one of claims 28-34, wherein the second exogenous polynucleotide sequence can be 1 bp to about 10 kb or longer.

36. The genome editing system according to any one of claims 1-35, wherein the first target sequence, the second target sequence, the first exogenous nucleotide sequence and/or the second exogenous nucleotide sequence are associated with plant traits, such as agronomic traits, whereby insertion of said first and/or second foreign nucleotide sequence results in said plants having altered (preferably improved) traits, such as agronomic traits, relative to wild-type plants.

37. The genome editing system according to any one of claims 1-36, wherein said plants include monocotyledonous plants and dicotyledonous plants, for example, said plants are crop plants including but not limited to wheat, rice, corn, soybean, Sunflower, sorghum, canola, alfalfa, cotton, barley, millet, sugar cane, tomato, tobacco, cassava and potato.

38. A method for producing a genetically modified plant, the genetically modified plant comprising a site-directed insertion of an exogenous nucleotide sequence, the method comprising introducing the genome editing system according to any one of claims 1-37 into at least one of said plants.

39. The method of claim 38, further comprising screening said at least one plant for plants having the desired insertion of the exogenous nucleotide sequence.

40. The method of claim 38 or 39, wherein the genome editing system is introduced into a plant by a method selected from the group consisting of particle gun method, PEG-mediated protoplast transformation, Agrobacterium-mediated transformation, plant virus-mediated transformation, Pollen tube passage method and ovary injection method.

41. The method of any one of claims 38-40, said introducing comprising transforming said genome editing system into an isolated plant cell or tissue, and then regenerating said transformed plant cell or tissue into an intact plant.

42. The method of any one of claims 38-40, said introducing comprising transforming said genome editing system into a specific part on a whole plant, such as a leaf, a shoot tip, a pollen tube, a young ear or a hypocotyl.

43. The method of any one of claims 38-42, further comprising treating (such as culturing) the plant cell, tissue or complete plant that has been introduced into the genome editing system at an elevated temperature, the elevated temperature is for example 37°C.

44. The method of any one of claims 38-43, wherein components of the genome editing system are simultaneously introduced into the plant.

45. The method of any one of claims 38-43, comprising introducing the genome editing system of any one of claims 28-37 into at least one of said plants, and comprising the steps of:
1) transforming components i)-iv) of the genome editing system into isolated plant cells or tissues, and obtaining the first exogenous nucleotide inserted into a recognition site (RS) comprising one or more recombinases sequence of plant cells or tissues;
2) transforming component v) of the genome editing system into the plant cell or tissue obtained in step 1), thereby obtaining a plant cell or tissue comprising the inserted second exogenous polynucleotide sequence; and
3) regenerating whole plants from the plant cells or tissues obtained in step 2).

46. The method of any one of claims 38-45, wherein the exogenous nucleotide sequence is inserted into a safe harbor site in the plant genome, the safe harbor site being in the plant genome
1) at least 5kb away from a protein coding region;
2) at least 30kb away from a miRNA coding region;
3) at least 20kb away from lncRNA coding region;
4) at least 20kb away from a tRNA coding region;
5) at least 5kb away from a promoter and/or enhancer;
6) at least 20kb away from a LTR repeat;
7) at least 200bp away from a non-LTR repeat; and
8) at least 10kb away from the centromere.

47. The method of claim 46, wherein said plant is rice, and said safe harbor loci are selected from: the loci shown in Table 1 or Table 2.

48. A method of producing a genetically modified plant comprising a site-directed insertion of an exogenous nucleotide sequence, the method comprising inserting the exogenous nucleotide sequence into a safe harbor in the plant genome point, the safe harbor site is in the plant genome
1) at least 5kb away from a protein coding region;
2) at least 30kb away from a miRNA coding region;
3) at least 20kb away from lncRNA coding region;
4) at least 20kb away from a tRNA coding region;
5) at least 5kb away from a promoter and/or enhancer;
6) at least 20kb away from a LTR repeat;
7) at least 200bp away from the non-LTR repeat; and
8) at least 10kb away from the centromere.

49. The method of claim 48, wherein said plant is rice, and said safe harbor loci are selected from: the loci shown in Table 1 or Table 2.
